Europäisches Patentamt

European Patent Office

Office européen des brevets

(11) Publication number:

**0 302 435**
**A1**

## EUROPEAN PATENT APPLICATION

(21) Application number: 88112480.4

(51) Int. Cl.⁴: **G01N 33/569 , G01N 33/50**

(22) Date of filing: 01.08.88

(30) Priority: 31.07.87 US 80319
25.04.88 US 185733

(43) Date of publication of application:
08.02.89 Bulletin 89/06

(84) Designated Contracting States:
AT BE CH DE ES FR GB GR IT LI LU NL SE

(71) Applicant: NEORX CORPORATION
410 West Harrison Street
Seattle Washington 98119(US)

(72) Inventor: Morgan, Alton C., Jr.
8615 - 187th Place, S.W.
Edmonds, Washington 98020(US)
Inventor: Graves, Scott S.
10904 Circle Drive
Bothell Washington 98011(US)

(74) Representative: Brown, John David et al
FORRESTER & BOEHMERT
Widenmayerstrasse 4/I
D-8000 München 22(DE)

(54) Method for augmenting antibody-dependent cellular cytotoxicity.

(57) The present invention provides methods of in vitro assay for determining a time of optimal in vivo augmentation of an antibody-dependent, cell-mediated cytotoxicity (ADCC) response in a tumor bearing patient that has received one or more lymphokines. Methods for ex vivo activation of polymorphonuclear leukocytes (PMNs) for use in ADCC therapy mediated by monoclonal antibody are also disclosed. In addition, the invention describes the use of an effective amount of one or more activators capable of stimulating effector cells for ADCC.

EP 0 302 435 A1

# METHOD FOR AUGMENTING ANTIBODY-DEPENDENT CELLULAR CYTOTOXICITY

## TECHNICAL FIELD

The present invention relates generally to a method for treating tumor-bearing patients, and in particular, to methods of assaying in vivo augmentation of antibody-dependent, cell-mediated cytotoxicity (ADCC). The present invention also discloses a method for augmenting ADCC mediated by polymorphonuclear leukocytes PMNs) and monoclonal antibodies, which involves preincubating PMN effector cells with a lymphokine in order to optimize the cytotoxic response.

## BACKGROUND ART

Immunological approaches to cancer therapy have gained prominence in recent years. For instance, the development of monoclonal antibodies provided a means for specific targeting of drugs or toxins to tumor cells. Another therapeutic immuno-logical approach recently recognized involves augmentation or enhancement of the host immune response to cancer or tumor cells. A variety of host cells and factors are involved in the host's immune response to tumor cells, and the interaction between these various components is believed to serve a primary role in anti-tumor immunity.

One element of the host immune system is lymphoid cells. At the present time, several different types of lymphocytes have been identified and characterized. Those designated as "B cells" are involved in the production of antibody molecules which bind to a specific antigen. Yet another type of lymphocyte is the T cell, which also can be specific for a given antigen. T cells have been further subdivided by function into cytotoxic T cells, which are known to bind to and kill certain target cells; suppressor T cells, which are known to act to inhibit the host immune response at a time after immune stimulation; and helper T cells, which are known to be necessary for B cell differentiation and for cytolytic T cell responses.

Another subpopulation of lymphoid cells, designated "NK" or "natural killer" cells, are known to demonstrate spontaneous cytotoxic reactivity against a variety of tumor cells. NK cells have been associated with a morphologically identifiable subpopulation of cells called "large granular lymphocytes" (LGL), which represent about 5% of blood peripheral mononuclear cells (T. Timonen et al., J. Exp. Med. 153: 569, 1981). NK cells may be responsible for the host's natural resistance to tumor cells through immune surveillance, which is the host's early detection system for abnormal or malignant cells (G. Trinchieri and B. Perussia, Lab. Invest. 50: 489, 1984). The reactivity of NK cells against tumor cells can be augmented in vitro or in vivo by exposure to various interferons (J.R. Ortaldo et al., J. Immunol. 138: 3566, 1987).

Cancer therapy has recently focused on enhancement of the host's own immune response. In early protocols, heterogeneous vaccines were administered to cancer patients in order to stimulate the patient's immune system (R.P McCabe et al., J. Natl. Cancer Inst. 60: 773, 1978). However, in practice, vaccine augmentation has not proven efficacious. One problem with vaccine augmentation studies is that they were performed on patients with high tumor burdens. Multiple animal model studies have demonstrated that boosting of a host's endogenous immune response is inadequate to reduce large tumor burdens. Therefore, the vaccine augmentation studies as designed had a small probability of successful enhancement of the body's own defense system.

A more promising therapeutic approach, which has been termed "adoptive immunotherapy," also features enhancement of an individual patient's immune response to tumor cells. The prototype procedure was described by S.A. Rosenberg and W.D. Terry, Adv. Cancer Res. 25: 323, 1977, and utilized expansion of the patient's anti-tumor lymphocytes in vitro prior to reinfusion. However, an initial obstacle to the feasibility of this immunotherapeutic approach was the inability to generate large numbers of the patient's immune cells in vitro. The discovery and production of T cell growth factor (interleukin 2; IL-2) permitted in vitro expansion of a patient's own lymphocytes.

Several years later, Rosenberg described the lymphokine-activated killer (LAK) cell phenomenon (S.A. Rosenberg, J. Natl. Canc. Inst. 75: 595, 1985), which took advantage of not only the growth promotion of IL-2, but also its differentiation properties in inducing cytotoxic antitumor lymphocytes from a patient's own cells. According to Rosenberg's procedure, a large number of the patient's peripheral blood lymphocytes are isolated and cultured with IL-2 in large-scale tissue culture vessels. IL-2 promotes growth and activation of these lymphocytes, enhancing their ability to kill tumor cells as determined by in vitro cytotoxicity assays. Rosenberg's procedure of LAK therapy in human patients showed that tumor regression was optimally induced when both LAK cells and IL-2 were infused into the patient simultaneously.

More recently, a new variation of adoptive immunotherapy has been described (S.A. Rosenberg et al., Science 233: 1318, 1986). A lymphoid cell population was obtained from tumor-bearing patients that was 50-100 times more effective than LAK cells. This population consisted of tumor-infiltrating lymphocytes (TIL), and, as with LAK immunotherapy, the TIL cells were expanded in vitro upon exposure to IL-2. Optimal TIL therapy is dependent upon host immunosuppression at the time of infusion.

Adoptive immunotherapy utilizing either LAK or TIL cells suffers from several disadvantages. The first drawback is that effective tumor treatment is generally accompanied by frequent and severe side effects or toxicity. It is not clear whether the toxicity is attributable to the infusion of activated lymphocytes, which exhibit some nonspecific targeting to normal tissues, or to the concomitant administration of IL-2 (which is used to maintain the infused lymphocytes in an activated state). Several reports have suggested that LAK cells recognize and kill normal cells (P.M. Sondel et al., J. Immunol. 137: 502, 1986; B. Loveland et al., Immunol. 59: 159, 1986), in contrast to Rosenberg's original report that LAK cells will lyse only fresh tumor targets but not noncultivated normal cells (M.T. Lotze et al., Canc. Res. 41: 4420, 1981; E.A. Grimm et al., J. Exp. Med. 155: 1823, 1982). An additional disadvantage of the Rosenberg LAK protocol is the enormous cost of in vitro expansion and activation of each patient's lymphocytes. The calculated cost of culturing the patient's lymphocytes for 4-6 days prior to reinfusion is approximately 10,000 per course (S.A. Rosenberg, Technol. Perspective--The Wilkerson Group, Inc. 1: 1, 1986). Yet another drawback stems from the need to culture the patient's lymphocytes in the presence of a human AB serum supplement. The use of human blood products substantially increases the risk of viral infections, such as hepatitis and AIDS.

Attempts have been made to infuse IL-2 directly into patients, on the theory that the patient's lymphocytes could be activated endogenously. This technique would avoid the costs and risks associated with large-scale in vitro culture of the patient's cells, as required by the Rosenberg procedure. However, several suggest that infusion of IL-2 for endogenous activation of lymphocytes is not therapeutically feasible.

The first factor limiting optimal endogenous activation with IL-2 is illustrated by a study by A.B. Reske-Kunz et al., Scand. J. Immunol. 23: 693, 1986. This study examined the interaction of IL-2 with IL-2 receptors expressed on lymphoid cells, and found that optimal effector cell cytolysis requires high IL-2 receptor occupancy, a condition not achievable in vivo due to the short serum half-life and dose-limiting toxicity of IL-2. Corollary studies have demonstrated the existence of high and low affinity receptors for IL-2 on activated cells, with the high affinity receptors mediating most of the biological activities ascribed to IL-2 (R.J. Robb and C.M. Rusk, J. Immunol. 137: 142, 1986; M. Fujii et al., J. Immunol. 137: 1552, 1986). In addition, IL-2 preactivation has been shown to reduce sensitivity of T lymphocytes to subsequent exposure to higher doses of IL-2 (J.D. Ashwell et al., J. Immunol. 137: 2572, 1986). The insensitivity to increased amounts of IL-2 was believed to be due to a requirement for a greater number of high affinity IL-2 receptors to be occupied to achieve a given biological response.

Most cytolytic activity of activated lymphocytes is probably mediated through cytolytic proteins present in granules. These granules are believed to mediate LAK killing (P.A. Henkart et al., J. Immunol. 137: 2611, 1986). Optimal granule formation often requires prolonged exposure to IL-2 in vitro. Thus in vivo formation of granules would require levels of IL-2 that probably are not currently achievable.

All these data suggest that upon stimulation of LAK cell precursors with IL-2, increasing doses of IL-2 would be required to further increase differentiation of the precursors to optimally activated LAK cells in vitro or in vivo. The requirement for increasing doses of IL-2 to elicit or maintain LAK populations in vivo would be hindered by pharmacokinetic difficulties in administering enough IL-2 in vivo to achieve a high degree of receptor occupancy. These difficulties include the short half-life of IL-2 upon administration in vivo (J.H. Donohue and S.A. Rosenberg, J. Immunol. 130: 2203, 1983), as well as the inherent toxicity of IL-2 in vivo. Accordingly, it may not be pharmacologically possible to stimulate a patient's lymphocytes in vivo to achieve optimum tumor cell killing.

Another immunological mechanism which may have implications for cancer therapy is antibody-dependent, cell-mediated cytotoxicity (ADCC). ADCC would utilize monoclonal antibody bound to tumor targets to direct the host's effector mechanisms through complement- or Fc receptor-mediated binding of leukocytes.

Recent studies have focused on the ability of mouse monoclonal antibodies to exhibit ADCC in conjunction with human immune components. In one study, anti-melanoma monoclonal antibody 9.2.27.54 (IgG2a) displayed minimal ADCC when assayed in the presence of human large granular lymphocytes (LGL) and human melanoma target cell lines (R.B. Herberman et al., in Monoclonal Antibodies and Cancer Therapy, R.A. Reisfeld and S. Sell, eds., 1985, Alan R. Liss, Inc., New York, NY, pp. 193-203; and J.R. Ortaldo et al., J. Immunol. 138: 3566, 1987). ADCC activity was improved by using a second monoclonal antibody MB3.6 (IgG3). In vitro ADCC of both MAbs (monoclonal antibodies) could be augmented by pretreatment of the LGL with lymphokines, such as interferon-alpha or IL-2.

Although increased tumor cell cytotoxicity was observed in the presence of the IgG3 monoclonal antibody (MAb), the mechanism of ADCC augmentation by MAb was unclear. Two alternatives were suggested: either the GD-3 glycolipid antigen recognized by MB3.6 was an especially relevant target structure for ADCC or the IgG3 subclass was more effective than the IgG2a subclass. In support of the former alternative, a second MAb, R24, directed to the same GD-3 glycolipid, which showed some clinical efficacy when administered to patients, was shown to bind to cross reactive antigen present on a subpopulation of T cells. The bi-arm binding between tumor cells and T cells could induce ADCC; or by enhancement of T cell proliferation, T cell infiltrates in tumors could be expanded and their efficacy increased (P. Hersey et al., Canc. Res. 46: 6083, 1986).

Some investigators have begun to examine augmentation of effector cells in vivo using mouse models. In one study, mice were injected with IL-2 prior to examination of ADCC and effector cell killing (S. Hinuma et al., Immunol. 59: 251, 1986). A serious drawback is that the effects of IL-2 on effector cell killing and ADCC were not measured on the same cell target, and ADCC was assessed using an IgG2a MAb. Levels of enhancement were very low because Hinuma et al. did not utilize an ADCC-effective MAb subclass. A second study utilized administration of IL-2 in combination with reinfusion of LAK cells to maintain an effector cell response in vivo in mice (M.Z. Papa et al., Canc. Res. 46: 4973, 1986). Only minimal boosting of effector functions, including ADCC, was observed. Yet another publication indicated that cyclophosphamide pretreatment, as well as IL-2-activated effector cells and IL-2 administration, were required for optimal treatment of metastases (S.A. Rosenberg et al, Science 233: 1318, 1986). The latter two studies point out a significant defect in both in vitro and in vivo experiments with lymphokines and murine effector cell-mediated killing -- the lack of correlation between in vitro and in vivo responses. This defect is further demonstrated by data showing that IL-2-stimulated T cells did not have significant in vitro cytotoxicity against target ovarian cancer cell lines, but upon injection in vivo produced pronounced anti-tumor effects (J.R. Ortaldo et al., Canc. Res. 46: 4414, 1986). The Papa et al. publication further noted that target cell cytolysis by LAK cells in vitro does not predict therapeutic efficacy against the same target cells in vivo.

The relation of activated effector cells and ADCC to cytotoxicity is unclear. Although in vitro experiments have investigated the role of ADCC in tumor killing, it is not presently known whether ADCC can be stimulated in vivo with the levels of IL-2 currently achievable in patients. The short serum half-life and toxicity of IL-2 limit the maximal in vivo level of IL-2 obtainable. It is further unclear whether conditions optimal for LAK cell generation are also optimal for ADCC. In addition, it is also unclear what the optimal type of MAb is to augment ADCC. The present invention significantly improves ADCC augmentation of host anti-tumor responses and further provides other related advantages.

## DISCLOSURE OF THE INVENTION

The present invention provides a method of assay for determining a time of optimal in vivo augmentation of an antibody-dependent, cell-mediated cytotoxicity (ADCC) response in a tumor-bearing patient. The inventive method comprises the steps of removing a basal effector cell sample from the patient to determine the basal of ADCC and NK or LAK cell activity of the patient; administering lymphokine to the patient in single or multiple doses; removing effector cells from the patient at predetermined time intervals after administration of lymphokine; assaying at each interval a first aliquot of the effector cells to establish a test level of ADCC and to determine the increase in ADCC above the basal level; assaying at each interval a second aliquot of the effector cells to determine the increase in NK or LAK cell activity above the basal level, establishing the differential between antibody- (Ab) and non-Ab-directed cell killing and thereby determining a time of optimal in vivo activation of the effector cells for an ADCC response in order to determine the optimal period for antibody infusion.

The basal effector cell sample of the foregoing method comprises multiple samples removed over a period of time. The step of determining the basal level of ADCC comprises the steps of combining the basal effector cell sample, an antibody, and target cells and detecting cytotoxicity by target cell lysis. Preferably, the antibody is a monoclonal antibody selected from the group consisting of murine IgG3, rat IgG2b,

chimeric (mouse/human) IgG₁, chimeric IgG₂, chimeric IgG₃, chimeric IgG₄, and heterohybrid monoclonal antibodies capable of bi-arm binding. Additionally, the lysis assay is selected from the group consisting of $^{51}$Chromium release, $^{111}$Indium release, and trypan blue exclusion. The target cells are cultured tumor cells that are antigenically similar to tumor cells from the patient. The lymphokine or combination of lymphokines is selected from the group consisting of interleukin-2, interleukin-4, interleukin-6, GM-CSF, G-CSF, M-CSF, and interleukin-3.

The step of determining a time of optimal activation comprises the steps of comparing at each time interval the test level of ADCC with or without exogenous lymphokine and the test level of LAK or NK activity to determine a difference between same test levels and identifying the time at which no further augmentation of ADCC occurs by addition of exogenous lymphokine or lymphokine combinations. Preferably, the preselected differences are calculated utilizing lytic units. The method may additionally comprise the step of administering cytoxan to the patient before or during lymphokine administration. The method may also additionally comprise the step of administering to the patient a therapeutically effective dose of ADCC-effective antibody directed against the patient's tumor after the step of determining a time of optimum in vivo activation of the effector cells. These antibodies are preferably selected from the group of antibodies consisting of murine IgG₃, rat IgG₂ᵦ, chimeric (mouse/human) IgG₁, chimeric IgG₂, chimeric IgG₃, chimeric IgG₄, and heterohybrid monoclonal antibodies capable of bi-arm binding.

The method may also comprise the additional step of infusing the lymphokine in dosages adequate to maintain optimum in vivo activation of the effector cells for a predetermined period of time after the step of determining the time of optimum in vivo activation of the effector cells for an ADCC response.

In another preferred embodiment, the present invention includes a method for in vivo treatment of a tumor-bearing patient comprising the steps of administering single or multiple doses of one or more lymphokines to the patient; determining a time of optimal in vivo activation of effector cells for an ADCC response; and infusing an effective ADCC antibody in doses adequate to localize the tumor and to initiate an anti-tumor response. The step of determining optimal in vivo activation of this treatment method incorporates the method disclosed above.

Yet another aspect of the present invention discloses a method for augmenting ADCC mediated by polymorphonuclear leukocytes (PMNs) and monoclonal antibodies comprising the steps of removing a leukocyte-containing sample from a tumor-bearing patient; separating a PMN fraction from the leukocyte-containing sample; incubating the PMN fraction with an ADCC-stimulatory amount of activator, thereby producing activated PMN effector cells; administering a therapeutic amount of an ADCC-effective monoclonal antibody to the tumor-bearing patient, thereby localizing the monoclonal antibody at the tumor; and infusing the activated PMN effector cells into the tumor-bearing patient, the activated PMN effector cells and the monoclonal antibody thereby initiating an anti-tumor response.


## BRIEF DESCRIPTION OF THE DRAWINGS


Figure 1 demonstrates in vitro augmentation of ADCC and NK cytolysis upon exposure of normal donor lymphocytes to low doses of IL-2 (1-10 U/ml) for 3 hours. Shown is chromium-51 release at various effector to target cell ratios. The level of killing with a murine IgG₁ MAb plus effectors is the same as with effector cells only. The ADCC MAb is a murine IgG₃ (NR-CO-04) and the target cell line is the colon carcinoma LS-180.

Figure 2 compares ADCC and LAK killing when normal lymphocytes are incubated in high doses (100-1000 U/ml) of IL-2 for a 6-day pre-exposure (typical LAK cell protocol) before testing of antibody- or non-antibody-mediated killings.

Figure 3 illustrates the IL-2 dose dependence of augmentation of ADCC and activation of NK killing with 3 hours of pre-exposure. Testing was done at an effector: target cell ratio of 50:1.

Figure 4 presents a clinical protocol for treating patients with IL-2 and cytoxan (200 mg/kg).

Figure 5 shows in vivo augmentation of one patient's ADCC response upon infusion of IL-2 according to the protocol of Figure 4, as demonstrated with ex vivo testing.

Figure 6 assesses the maximal ADCC response achievable by adding exogenous IL-2 to patient lymphocytes removed during IL-2 therapy. The difference in the curves shows what level of ADCC augmentation can be achieved with further IL-2 administration.

Figure 7 depicts PMN-ADCC of LS-180 target cells mediated by IgG₁ ( ◇——◇ ); by IgG₁ following a 1 h preincubation of PMNs with GM-CSF (500 U/ml) ( ■——■ ); by IgG₃ ( x - x); or by IgG₃ following a 1 h preincubation of PMNs with GM-CSF ( □——□ ).

Figure 8 shows the titration of GM-CSF with respect to PMN-ADCC and non-specific cytotoxicity. PMN were treated with different concentrations of GM-CSF for 1 h before assaying cytotoxic activity against LS-180 target cells (stipled bar); ADCC activity using NR-CO-02 monoclonal antibody and PMNs treated with GM-CSF (solid striped bars); or ADCC activity using NR-CO-04 monoclonal antibody and PMNs treated with GM-CSF (open striped bars).

Figure 9 demonstrates the kinetics of GM-CSF activation of PMNs. Control resting PMNs were either washed (heavy striped bar) or not washed (light striped bar) prior to addition of antibody and target cells. Alternatively, PMNs were treated for various periods of time with 100 U/ml GM-CSF and either washed (solid bar) or not washed (stipled bar) before addition of antibody and target cells.

Figure 10 presents the effect of in vivo administration of GM-CSF to M. fasicularis. Monkeys were assessed for blood cell profiles 1 and 8 days after continuous infusion of GM-CSF (Fig. 10a). Ficoll-separated PMNs were assessed for ability to mediate ADCC with NR-CO-04 antibody and LS-180 target cells on days 1 and 8 (Fig. 10b).

## DETAILED DESCRIPTION OF THE INVENTION

Prior to setting forth methods related to NK- (LGL-) mediated ADCC, it may be helpful to an understanding thereof to set forth definitions of certain terms to be used hereinafter.

### Lymphokine:

A protein or organic compound capable of regulating an immunological response through binding to specific receptors (e.g., IL-2, IL-3, IL-4, IL-6, M-CSF, G-CSF, GM-CSF). "Lymphokine" includes peptides derived from native lymphokines and drug molecules that possess functional properties analogous to those of native lymphokines.

### Effector cell:

Lymphoid cells of granulocyte, monocyte or lymphoid lineage capable of mediating lysis of target cells.

### Target cell:

Typically, cultured or fresh human tumor cells, which may be lysed by effector cells alone or by a combination of bound antibody and effector cells.

### NK cell:

A high-density (Percoll) lymphocyte cell having a large granular lymphocyte (LGL) phenotype and defined by cell surface markers Leu-M1 and OKM-1. NK cells are capable of mediating lysis of certain target cells, e.g., K562.

### Activated NK cell:

A large granular lymphocyte population exposed to low doses (1-50 U/ml) of lymphokine for short periods (1-3 hours). Activated NK cells are capable of mediating enhanced cytolysis of both NK-sensitive and NK-resistant targets.

## LAK cell:

Lymphokine-activated killer cells induced by exposure to high doses of lymphokine (50-1000 U/ml) for extended periods (2-6 days), in the absence of any antigenic stimulation. LAK cells are cytotoxic to both NK-sensitive and NK-resistant tumor target cells.

## ADCC:

Antibody-dependent, cell-mediated cytotoxicity. ADCC features binding of an effector cell to a target cell through the bridge created by an antibody molecule. The antibody may bind to an antigen expressed on the surface of the target cell and to an Fc or complement receptor on the effector cell. Alternatively, the same antibody may bind both the target and effector cell through bi-arm binding. The release of cytotoxic molecules from the effector cell in close proximity to the target cell may result in target cell cytolysis.

## ADCC-effective antibody:

Those antibodies belonging to the group consisting of: murine $IgG_3$, murine $IgG_{2a}$, rat $IgG_{2b}$, chimeric (mouse/human) $IgG_1$, chimeric $IgG_2$, chimeric $IgG_3$, chimeric $IgG_4$, and heterohybrids thereof.

## AUGMENTATION OF NK-MEDIATED ADCC

As noted above, immunotherapy for treatment of cancer has received much attention recently. Adoptive immunotherapy has progressed to the stage of clinical trials, but the procedure is fraught with certain disadvantages, as discussed above. The present invention offers an immunotherapeutic approach to cancer therapy that avoids the risks and expense of currently employed protocols.

One advantage of the present invention is that it utilizes augmentation of the host ADCC response and is not dependent on optimal stimulation of the host's LAK/effector cell population for anti-tumor activity. in vitro experiments have demonstrated that host ADCC responses, especially those mediated through LGL or other Fc receptor-bearing cells, are augmented by lower doses of IL-2 than are required for activation of LAK cells. Another advantage is that the doses of IL-2 required for ADCC augmentation in vivo are pharmacologically tolerable, and provide optimal stimulation of ADCC upon in vivo administration of lymphokine. Improved specificity and potency of antibody-mediated killing of tumor cells are additionally provided by the present invention.

The present invention describes a method for in vivo augmentation of an antibody-dependent, cell-mediated cytotoxicity (ADCC) response in a tumor-bearing patient, comprising removing a basal effector cell sample from the patient; determining a basal level of ADCC and NK cell activity, utilizing said sample; administering a single or multiple doses of lymphokine to the patient; removing effector cells from the patient at intervals after administration of lymphokine; assaying at each interval an aliquot of the effector cells to establish the level of augmentation of ADCC; assaying at each interval a second aliquot of the effector cells to determine the increase in NK/LAK cell activity above the basal level; determining a time of optimal in vivo activation of the effector cells for an ADCC response, e.g., the point after lymphokine infusion at which the differences in the ADCC and NK/LAK effector-to-target-cell titration curves are maximally different; and infusing the lymphokine in dosages adequate to maintain the optimal in vivo activation of the effector cells for ADCC for a predetermined period of time.

In addition, the present invention provides for infusion of an ADCC-effective MAb in single or multiple doses in a range of 10-500 mg per administration. Antibody treatment at the time of optimal ADCC augmentation may be limited to a course of 10-12 days, due to the ability of lymphokine to enhance host antiglobulin responses to the administered antibody. In this regard, cessation of lymphokine administration during antibody treatment or use of mouse/human chimeras may help reduce the indcidence and severity of the antiglobulin response.

According to the present invention, one or more basal effector cell samples are removed from the patient prior to administration of lymphokine. In a preferred embodiment, the basal sample(s) are frozen in liquid nitrogen and tested in the same assay as samples removed during lymphokine administration. In this way, the basal level of ADCC can be compared to augmented levels in the most non-ambiguous way. A preferred embodiment comprises measuring ADCC of patient samples by combining Ficoll-Hypaque-isolated mononuclear cells, antibody, preferably of the murine $IgG_3$ subclass, and target cells which have been selected for low levels of NK killing, and detecting cytotoxicity with a target cell lysis assay. Preferred target cell lysis assays include $^{51}Cr$ release, $^{111}In$ release, and trypan blue exclusion.

The administered lymphokine is preferably given to the patient by intravenous, slow infusion to maximize serum half-life. A particularly preferred lymphokine in this regard is interleukin-2 (IL-2). It will be evident to one skilled in the art that other lymphokines may be suitable within the present invention for activating cell populations for augmented ADCC. Such lymphokines may include GM-CSF, G-CSF, M-CSF, IL-3, IL-4, and IL-6, which may be used alone or in any combination with one or more of these lymphokines. A particularly preferable combination is IL-2 in combination with IL-6 (B cell-stimulating factor 2, interferon B2), which has been shown to be a synergistic combination for T cell proliferation, as well as IL-2 with IL-4 (Lichtman et al., Proc. Natl. Acad. Sci. USA 84: 824, 1987; Severinson et al., Eur. J. Immunol. 17: 67, 1987). In a further preferred embodiment, cytoxan in the 150-400 mg/kg range is preadministered to the patient at selected intervals 24 hours before lymphokine to selectively abrogate immunosuppressive effects of T suppressor cells.

The step of determining a time of optimal in vivo activation of effector cells for an ADCC response preferably comprises the steps of comparing, at each time interval, the test levels of a patient's ADCC activity and NK and LAK cell activity by comparison to normal donor lymphocytes or to the patient's own basal samples to determine the level of augmentation with lymphokine administration. By examining non-stimulated and in vivo-stimulated samples compared to the in vivo-stimulated sample with exogenous IL-2 added in vitro (see Example 6), one can determine if ADCC is optimally stimulated and whether antibody infusion can be started.

## AUGMENTATION OF PMN-MEDIATED ADCC

The primary function of polymorphonuclear leukocytes PMNs) is the protection of a host against invasion by pathogenic organisms. This protective process is mediated by release of reactive oxygen metabolites (such as hydrogen peroxide and superoxide), cytotoxic enzymes (such as myeloperoxidase) or halides.

Another function of PMNs is mediation of nucleated cell cytolysis through the mechanism of antibody-dependent cellular cytotoxicity (ADCC). ADCC may be defined or measured by standard in vitro assays, such as $^{51}Cr$ release from target cells. This in vitro measurement of ADCC correlates with the ability of an antibody to induce an in vivo inflammatory response at a targeted tissue.

Nucleated cell targets of ADCC include those of neoplastic origin. The ADCC mechanism of killing may be mediated by several different types of effector cells, including large granular lymphocytes (LGL) and monocytes/macrophages, as well as PMNs. In addition, complement may have a direct cytolytic effect on target cells in the presence of anti-target cell antibody, or may act through receptors on effector cells to produce indirect cytolysis of target cells. It is desirable to stimulate and augment the ADCC capacity of various effector cells, in order to optimize their cytotoxic activity toward particular target cells.

The ADCC process involves three components: effector cells, antibody and target cells. PMN-mediated ADCC of nucleated neoplastic target cells requires Fc receptor binding of an anti-tumor antibody to PMN effector cells. The antigen-combining region of the anti-tumor antibody binds to a tumor antigen of the target cell, thus forming a "bridge" between the effector and target cell. PMN-mediated cytotoxicity of the target cells may be then effected through either of two distinct mechanisms: (1) neutrophil production of oxygen-reactive species or (2) granule exocytosis of cytolysin-like molecules.

PMN-mediated ADCC of tumor target cells has previously been examined using polyclonal anti-tumor antibodies. In theory, murine monoclonal antibodies hold great promise as potential mediators of ADCC. For instance, monoclonal antibodies provide an unlimited, homogeneous supply of very specific anti-tumor agents. To be useful as therapeutic agents, these monoclonal antibodies need to interact with human effector cells (PMNs) to produce cytolysis of human tumor target cells.

The present invention discloses that monoclonal antibodies alone are very poor mediators of PMN-ADCC when tested in a standard ADCC assay with human effector and target cells. The claimed invention overcomes this disadvantage and provides a method for augmenting PMN-mediated ADCC of tumor target cells using monoclonal antibodies. This augmentation is achieved by preincubating the PMN effector cells with a lymphokine ex vivo prior to combination of the PMNs with monoclonal antibody and target cells in vivo. Alternatively, a lymphokine may be administered to a tumor-bearing patient in vivo, in an amount effective for ADCC augmentation, prior to infusion of an anti-tumor monoclonal antibody.

One embodiment of the present invention discloses a method for in vivo treatment of a tumor-bearing patient, comprising the steps of:

removing a leukocyte-containing sample from a tumor-bearing patient;

separating a polymorphonuclear leukocyte (PMN) fraction from the leukocyte-containing sample;

incubating the PMN fraction with an ADCC-stimulatory amount of activator, thereby producing activated PMN effector cells;

administering a therapeutic amount of an ADCC-effective monoclonal antibody to the tumor-bearing patient, thereby localizing the monoclonal antibody at the tumor; and

infusing the activated PMN effector cells into the tumor-bearing patient, the activated PMN effector cells and the monoclonal antibody thereby initiating an anti-tumor response.

Blood and bone marrow samples are exemplary of a leukocyte-containing sample of the present invention. Any of the well-known standard methods for separating PMNs from a leukocyte-containing sample may be used within the claimed methods.

Significant enhancement of a monoclonal antibody-mediated PMN-ADCC response is observed when PMN effector cells are preincubated with a lymphokine or PMN activator. Within the present invention, the term "activator" will be inclusive of both lymphokines and PMN activators. Preferred activators include granulocytemonocyte colony stimulating factor (GM-CSF), granulocyte colony stimulating factor (G-CSF), gamma-interferon (gamma-IFN), fMET-Leu-Phe peptide (fMLP), and phorbol myristate acetate (PMA), with GM-CSF a particularly preferred lymphokine.

In addition, protein kinase activators may be used within the claimed methods. Further, the calcium ionophore A23187 may be used synergistically with PMA for PMN activation.

Combinations of activators may also be used advantageously within the present invention. For instance, lymphokines other than GM-CSF or protein kinase activators may be used in combination with GM-CSF to induce activated PMNs that maintain augmented ADCC activity for an extended time. The incubation of PMN effector cells with combinations of lymphokines may involve addition of the lymphokines in sequence or simultaneously.

Preferred concentrations of activator for ex vivo incubation with PMNs range from about 0.1 U/ml to about 1000 U/ml, with concentrations from about 10 U/ml to about 500 U/ml particularly preferred. Obviously, the amount of lymphokine employed may be dependent on the particular combination of lymphokine and effector cell: antibody: target cell used for ADCC killing of target cells.

Suitable monoclonal antibodies for use within the present invention include murine monoclonal $IgG_3$ antibodies; chimeric monoclonal antibodies (e.g., mouse/human chimeras), such as chimeric $IgG_1$ and chimeric $IgG_3$; and human $IgG_1$ and $IgG_3$ monoclonal antibodies. Heterohybrids or bivalent antibodies capable of binding both target and effector cells through Fab or antigen-combining regions are also useful within the claimed methods. A preferred monoclonal antibody in this regard is a murine $IgG_3$ monoclonal antibody directed against carcinoma cells, with monoclonal antibody NR-CO-04 particularly preferred. Obviously, monoclonal antibodies (murine or chimeric) having other tumor target cell specificities would also be suitable for use within the claimed invention. For instance, a pan carcinoma monoclonal antibody that reacts with a proper antigen on a target tumor cell and binds through its Fc domain to PMNs so as to stimulate PMN-ADCC may be used within the present invention.

The present invention utilizes a standard ADCC assay. For instance, the target cell lysis assay used to measure an ADCC response may include $^{51}$Cr release, [111]Indium release and trypan blue exclusion.

The first embodiment of the invention may further include, after the step of incubating PMNs with lymphokine, the further step of removing the lymphokine from the PMNs.

The amount of ADCC-effective monoclonal antibody administered to the patient preferably is sufficient to localize at and bind to antigen-positive cells within the target tissue or organ. The monoclonal antibody may be given in a single dose, in multiple doses or by continuous infusion.

A second embodiment of the present invention is a method for in vivo treatment of a tumor-bearing patient, comprising the steps of:

removing a leukocyte-containing sample from the tumor-bearing patient;

separating a polymorphonuclear leukocyte (PMN) fraction from the leukocyte-containing sample;

incubating the PMN fraction with an ADCC-stimulatory amount of activator, thereby producing activated PMN effector cells;

sensitizing the activated PMN effector cells with an ADCC-effective amount of a monoclonal antibody directed against the patient's tumor, thereby forming an activated PMN-monoclonal antibody complex; and

infusing the activated PMN-monoclonal antibody complex into the tumor-bearing patient, thereby initiating an anti-tumor response.

9

A third embodiment of the claimed invention involves a method for in vivo treatment of a tumor-bearing patient, comprising the steps of:

infusing an activator into the tumor-bearing patient in an amount sufficient for in vivo activation of PMN effector cells; and

administering a therapeutic amount of an ADCC-effective monoclonal antibody to the tumor-bearing patient, thereby localizing the monoclonal antibody at the tumor and initiating an anti-tumor response.

A fourth embodiment of the present invention discloses a method for in vivo treatment of a tumor-bearing patient, comprising the steps of:

infusing a first activator into the tumor-bearing patient in an amount sufficient for in vivo proliferation of PMN effector cells, thereby inducing leukocytosis in the patient;

infusing a second activator into the tumor-bearing patient, at a time subsequent to leukocytosis induction, in an amount sufficient for in vivo activation of the PMN effector cells; and

administering a therapeutic amount of an ADCC-effective monoclonal antibody to the tumor-bearing patient, thereby localizing the monoclonal antibody at the tumor and initiating an anti-tumor response.

A fifth embodiment of the claimed invention describes a method for in vivo treatment of a tumor-bearing patient, comprising the steps of:

infusing a first activator into the tumor-bearing patient in an amount sufficient for in vivo proliferation of PMN effector cells, thereby inducing leukocytosis in the patient;

removing a leukocyte-containing sample from the tumor-bearing patient;

separating a polymorphonuclear leukocyte (PMN) fraction from the leukocyte-containing sample;

incubating the PMN fraction with an ADCC-stimulatory amount of a second activator, thereby producing activated PMN effector cells;

infusing the activated PMN effector cells into the tumor-bearing patient; and

administering a therapeutic amount of an ADCC-effective monoclonal antibody to the tumor-bearing patient, thereby localizing the monoclonal antibody at the tumor and initiating an anti-tumor response.

A sixth embodiment of the present invention describes a method for ex vivo activation of polymorphonuclear leukocytes (PMNs) for use in antibody-dependent cellular cytotoxicity (ADCC) therapy mediated by monoclonal antibody, comprising the steps of:

removing a leukocyte-containing sample from an appropriate donor;

separating a polymorphonuclear leukocyte (PMN) fraction from the leukocyte-containing sample; and

incubating the PMN fraction with an ADCC-stimulatory amount of activator, thereby producing activated PMNs for use in ADCC therapy in mediated by monoclonal antibody.

To summarize the examples that follow, Example I describes the cell lines, reagents, and assays used in determining lymphokine augmentation of NK-mediated and PMN-mediated ADCC. The in vitro data which support the feasibility of lymphokine augmentation of NK-mediated ADCC are presented in Example II. Results derived from in vivo infusion of lymphokine into patients and the subsequent in vitro assessment of NK-mediated ADCC augmentation are shown in Example III.

Example IV shows the effect of GM-CSF on PMN-ADCC, while Example V characterizes the antigen specificity of GM-CSF-augmented PMN-ADCC. Titration of the amount of GM-CSF required for PMN-ADCC is presented in Example VI; kinetics of GM-CSF augmentation is described in Example VII. GM-CSF activation and protein kinase C activation of effector cells are compared in Example VIII. Induction of leukocytosis after in vivo administration of GM-CSF to monkeys is illustrated in Example IX. Example X describes ex vivo activation of PMNs with GM-CSF for augmenting ADCC in a tumor-bearing patient.

The following examples are offered by way of illustration, and not by way of limitation.

EXAMPLE I

Cells, Reagents, and Assays

10

## A. Effector Cells for NK-ADCC

The augmentation of ADCC with low doses of IL-2 was first examined in vitro, using normal donor lymphocytes as effector cells. To prepare the effector cells, peripheral blood was collected in 15-ml heparinized tubes (Curtin Matheson Scientific, Inc., Tukwila, WA). An equal volume of 3% dextran was mixed with the blood end the mixture was incubated at room temperature for 30 min. The leukocyte-rich plasma layer was then removed. In a 50-ml conical tube, 12.5 ml Histopaque-1119 (Sigma Chemical Co., St. Louis, MO) was added, 12.5 ml lymphocyte separation medium (LSM; Organon Teknika, Durham, NC) was layered above, and 25 ml leukocyte-rich plasma was then layered on top. For mononuclear leukocyte preparation, peripheral blood was diluted 1:2 in phosphate-buffered saline (PBS) and 35 ml diluted blood was layered on top of 15 ml LSM. The conical tubes were centrifuged at 650 x g for 20 min at room temperature. The mononuclear layers were removed and placed in another 50-ml tube; each polymorphonuclear leukocyte layer was also removed to another 50-ml tube. Each of the removed cell layers was diluted to 50 ml with PBS, and the cells were centrifuged (mononuclear - 650 x g, 10 min, room temperature; PMNs - 250 x g, 5 min, room temperature). If the PMN layer displayed significant visible red blood cell contamination, the PMNs were resuspended in 5 ml deionized water for 15-30 sec, quickly diluted to 50 ml with PBS, and centrifuged at 250 x g, 5 min, room temperature. All pellets of the same cell type were combined, the preparations diluted to 50 ml with PBS and centrifuged according to the cell type, as noted above. The cell pellets were resuspended in assay medium [RPMI-1640 with L-glutamine (Whittaker MA Bioproducts, Walkersville, MD) containing 10% heat-inactivated fetal calf serum (FCS)], counted, and diluted to appropriate concentrations. The cell preparations were also assessed for viability by exclusion of 0.4% trypan blue.

## B. Effector Cells for PMN-ADCC

Peripheral blood polymorphonuclear leukocytes (PMNs) were obtained from healthy normal human donors. Blood (150 cc) was withdrawn by venipuncture in heparinized tubes and mixed with an equal volume of 3% dextran in phosphate-buffered saline (PBS). After sedimentation at 1 x g for 10 min at room temperature, the leukocyte-rich plasma was layered on top of lymphocyte separation medium (LSM, Organon Technica, Durham, NC). PMNs were purified by treating the PMNs and red blood cell (RBC) pellet with RBC lysing solution (0.800% w/v $NH_4Cl$, 0.1% w/v $KHCO_3$, 37.0 mg tetrasodium EDTA in 100 ml water at pH 7.3). After incubating approximately 5 min at room temperature, the volume was increased 3X with PBS and the cells washed twice by centrifugation. After resuspension of the cells in RPMI-5% FCS, the PMNs were used in cytotoxicity assays or were incubated with response modifiers. Cytochemical staining with enumeration by microscopy indicated that the PMNs were greater than 90% purified. Further, the purification technique does not result in activation of the PMNs.

For monkey studies, peripheral blood from cynomogolous monkeys (M. fasicularis) was diluted 1:4 with PBS and layered on top of 95% LSM. The preparations were then centrifuged at 100 x g for 20 min at room temperature. Monkey PMNs were purified according to the method described for human PMNs, and yielded PMNs of greater than 80% homogeneity as determined by cytochemical staining and enumeration by microscopy.

## C. Target Cells

The principle target cells used within the present invention are LS-180 cells, which are derived from a patient with adenocarcinoma of the colon. LS-174, LoVo and SW1-222, also of colon adenocarcinoma origin, and MDA-MB-484, a breast adenocarcinoma cell line, were also used to assess PMN-mediated cytotoxicity.

The target cells were maintained as adherent cultures in RPMI 1640 medium supplemented with 10% serum (8% Serum Plus, Hazelton Laboratories, Lenexa, KS, and 2% newborn calf serum, M.A. Bioproducts, Walkersville, MD), 2 mM L-glutamine and 1 mM sodium pyruvate (M.A. Bioproducts). The cultures were grown at 37° C in a humidified atmosphere of air with 5% $CO_2$.

When the target cell cultures reached confluency, the culture medium and floating cells were decanted, and the adherent cells were washed with 10 ml PBS. Target cells were released by either EDTA-mechanical

11

force or trypsin treatment prior to centrifugation at either 250 x g for 5 min or 150 x g for 10 min. The cell pellet was removed and diluted in 1 ml assay medium without FCS, and the cells were counted.

LS-180(T) designates LS-180 cells that have been passaged in nude mice. To obtain LS-180(T) cells, Nu Nu mice are injected subcutaneously with 2 x $10^6$ trypsin-released LS-180 cells in a 200μl volume of RPMI 1640. After tumors grow to a palpable size (approximately 7 days) they are surgically removed, separated by passage through a stainless steel wire mesh screen, and centrifuged at 150 x g for 10 min. The cell pellet is then washed and resuspended in RPMI containing 5% FCS, and the trypan blue-excluding cells are determined prior to labeling procedures.

## D. Reagents

Anti-adenocarcinoma monoclonal antibodies NR-CO-04 (IgG₃; directed against a tumor-associated glycolipid of colon cancer) and NR-CO-02 (IgG₁; directed against carcinoembryonic antigen) were used as standard reagents within the present invention. NR-LU-10 (IgG₂b) is a pancarcinoma monoclonal antibody.

Recombinant human GM-CSF was obtained from a COS cell transfectant (D. Metcalf et al., Blood 67:37-45, 1986). Retinal, phorbol myristate acetate (PMA) and A23187 were obtained from Sigma Chemical Co., St. Louis, MO.

## E. Target Cell Lysis Assay of NK-ADCC

For NK-ADCC cytotoxicity assays, an appropriate number of LS-180 target cells were labeled with 400-500 μl sodium $^{51}$Cr (1 mCi/ml; New England Nuclear, Boston, MA) for 2 h at 37°C, with gentle mixing every 15-30 min. The labeled cells were washed twice with 15 ml assay medium followed by centrifugation at 250 x g for 5 min at room temperature. The final pellet was resuspended in assay medium containing 10% FCS, counted, and diluted to 2 x $10^5$ viable cells/ml.

In a typical assay of IL-2 augmentation of NK-ADCC, 50 μl of an appropriate concentration of IL-2 was added to wells of a microtiter plate, except for those wells to be used for determination of total and spontaneous release of $^{51}$Cr from target cells. IL-2 was typically added at 100 U/ml or 10 U/ml final concentration. The wells that contained IL-2 also received 100 μl of an appropriate concentration of effector cells, and the plates were then incubated at 37°C for 3 h.

The wells to be used for determination of spontaneous $^{51}$Cr release received 200 μl assay medium; the wells to be used for determination of total $^{51}$Cr release received 200 μl 2% NP-40. Natural killer cell control wells consisted of three different effector: target (E:T) ratios (100:1, 50:1, 25:1) incubated with 50 μl assay medium. NK-ADCC-test wells consisted of 100:1, 50:1 and 25:1 E:T ratios incubated with 50 μl antibody diluted to various concentrations. For instance, with LS 180 target cells, murine IgG₃ MAb NR-CO-04 was added at 5 μg/well or 1 μg/well and served as a positive control; the negative control was IgG₁ MAb NR-CO-02, added at 5 μg/well or 1 μg/well. All wells then received 50 μl of an appropriate concentration of $^{51}$Cr-labeled target cells. The microtiter plate was centrifuged at 60 x g for 2 min at room temperature, and the plate was incubated at 37°C for 4 h.

After 4 h incubation, the microtiter plate was centrifuged at 250 x g for 5 min at room temperature, and 100 μl of supernatant was transferred from each well into a separate 12 x 75 mm glass tube. Each tube was then assayed for $^{51}$Cr release with a gamma counter.

All variables were generally examined in triplicate or quadruplicate. The means of replicate tubes were calculated, and the amount of NK-ADCC was determined according to the following formula:

$$\% \text{ lysis} = \frac{\text{experimental value--spontaneous release}}{\text{total release--spontaneous release}} \times 100$$

"Final percent lysis" was calculated by subtracting percent lysis values of the natural killer cell (NK) control wells from percent lysis values of the NK-ADCC-test wells.

The number of effector cells required to obtain a given percentage of lysis of target cells has been designated a "lytic unit." Within this example, 10% lysis of target cells was selected for calculation of lytic units. To calculate lytic units using three different E:T ratios, a semi-log linear regression is determined for the corresponding percent lysis values at the three different E:T ratios. From the known value for 10% lysis obtained by linear regression analysis, the lytic unit (the number of effectors required to obtain 10% lysis) is calculated. The number of lytic units per $10 \times 10^6$ effector cells is then expressed.

F. Target Cell Lysis Assay of PMN-ADCC

An appropriate number of target cells were labeled with 200 $\mu$Ci $^{51}$Cr ($^{51}$Cr-Na$_2$CrO$_3$; New England Nuclear, Boston, MA) for 2 h at 37° C, with gentle mixing every 15-30 min. The labeled cells were washed three times with 15 ml RPMI followed by centrifugation at 250 x g for 5 min at room temperature. The final pellet was resuspended in RPMI containing 5% FCS, counted, and diluted to an appropriate concentration.

In a typical assay, effector PMNs at different effector:target ratios were added to wells of a microtiter plate (Falcon Plastics, Oxnard, CA), except for those wells to be used for determination of total and spontaneous release of $^{51}$Cr from target cells. For GM-CSF augmentation, PMNs were generally treated within the microtiter plates by the addition of the lymphokine. The effector cells were either washed before the addition of antibody and target cells, or were left in the presence of lymphokine during the ADCC assay.

NR-CO-04 antibody was added to appropriate wells at a concentration of 1 $\mu$g/well (4 $\mu$g/ml), and the microtiter plate was then incubated at 37° C for 3 h. Target cells ($1 \times 10^4$ cells in 50 $\mu$l RPMI-5%FCS) were added to the appropriate wells; the microtiter plate was centrifuged at 60 x g for 2 min at room temperature and was incubated at 37° C for 4 h.

Wells used for determination of spontaneous $^{51}$Cr release received labeled target cells alone in 200 $\mu$l of RPMI-5% FCS; the wells used for determination of total $^{51}$Cr release received labeled target cells and 200 $\mu$l 1.0% NP-40.

After 4 h incubation, the microtiter plate was centrifuged at 250 x g for 5 min at room temperature, and 125 $\mu$l of supernatant was transferred from each well into a separate 12 x 75 mm glass tube. Each tube was then assayed for $^{51}$Cr release with a Beckman 5000 gamma counter.

PMN-ADCC assays were performed in triplicate, according to the procedure described in Example I.E., supra. The % lysis (or percent specific release) is indicative of the level of ADCC of target cells. Test release is the amount of release in wells containing effector cells and target cells.

G. Two Color Fluorescence Cytometry to Assess Activation State of Effector Cells

The level of in vitro or in vivo activation of an appropriate effector cell population may be determined using two color fluorescence cytometry. Molecules both on the effector cell surface and within the effector cell are important for enhanced ADCC activity following exposure of effector cells to activators. Antigens that may become functionally enhanced include LFA (lymphocyte function-associated antigens) involved in cytolytic and adhesion processes on a variety of cell types, and proteins present in granules of LGLs, PMNs and activated T cells. Examples of the latter type of protein include cytolysin, perforin and various proteases and esterases. In addition, cell surface proteins that bind complement fragments may also be functionally enhanced upon exposure to activators. Increased or decreased cell surface or intracellular expression of such antigens following in vivo exposure to an activator may signal an appropriate time for infusion of an ADCC-effective antibody so as to achieve optimal therapeutic effect. Expression of these antigens on a patient's effector cells before and after treatment with activators may be compared in order to determine the activation state of the patient's effector cells for ADCC-effectiveness.

For instance, IL-2-induced activation of NK(LGL) effector cells is monitored using FITC- and phycoerythrin-labeled monoclonal antibodies directed against cell surface antigens present on NK cells. Briefly, plastic-nonadherent mononuclear cells are separated from peripheral blood lymphocytes obtained from a tumor-bearing patient both before and after IL-2 therapy. The nonadherent cells are washed by centrifugation and resuspended in buffer (PBS + 1% BSA + 1% human IgG). The cells are washed and antibody specific for NK(LGL), such as NKH-1, is added. After incubation of the mixture at 4° C for 45 min, the cells are washed by centrifugation and FITC-labeled goat anti-mouse F(ab')$_2$ (designated "GAM") is added. Following incubation for 30 min at 4° C, the cells are washed and normal mouse Ig is added to block any unbound FITC-GAM. A second marker antibody, phycoerythrin-labeled OKM-1 (a monoclonal antibody directed against the C3b receptor), is then added and incubated for 30 min at 4° C. Alternatively, phycoerythrin-labeled anti-CD2, anti-LFA-1 or anti-LFA-3 monoclonal antibodies may be used to assess NK cell activation.

Flow cytometric analysis is performed on an Epics C cytometer, with the forward and 90° light scatter window gated on the mononuclear cell population. Compensation for red-green overlap is established using stained samples. Negative control samples are analyzed to determine a level of background fluorescence intensity. A single phycoerythrin-labeled OKM-1 stained sample is run in order to set a red fluorescence gate on positively stained cells. Test samples are then analyzed, collecting green fluorescence gated on OKM-1-phycoerythrin-positive cells. For assessment of intracellular marker antigens, cells are permeabilized with lysolecithin prior to staining with antibodies.

GM-CSF-induced activation of PMN effector cells may be monitored by a similar method using FITC- and phycoerythrin-labeled monoclonal antibodies directed against cell surface antigens present on PMN cells. An appropriate monoclonal antibody for detecting activated PMNs is Leu-M1, which recognizes an antigen associated with granulocytes and monocytes.

## H. Cytofluorometric Analysis

Cytofluorometric analysis was used to determine density of antigen on target cell lines (LS-180, LS-174, LoVo, SW1-222, MDA-MB 484) recognized by NR-CO-04 and NR-CO-02 monoclonal antibodies. Target cells were incubated at 4°C for 45 min in the presence of murine monoclonal antibodies (1 μg/ml) diluted in PBS containing 1.0% BSA and 0.1% Cohn Fraction II human immunoglobulin. After two washes by centrifugation, the cells were labeled for 30 min at 4°C with FITC-labeled F(ab')$_2$ goat anti-mouse antibody. The cells were washed and fluorescein equivalents (F.E. ratio) of untreated target cells; FITC goat antibody plus target cells; and murine monoclonal antibody plus FITC goat antibody plus target cells were determined by comparing labeled cells to a standardized preparation of fluorescein-labeled beads (Coulter Corp., Hialeah, FL) on an Epics C cytometer (Coulter Corp.). Net F.E. were determined by dividing the fluorescence intensity of the test sample by the fluorescence intensity of the control.

## I. Induction of PMN Leukocytosis in M. fasicularis Monkeys

GM-CSF was administered to age- and weight-matched M. fasicularis monkeys as an intravenous bolus of 50 μg/kg over a 1-2 min period, followed by a continuous intravenous drip infusion of 50 μg/kg day for 10 days. The injections were administered through an indwelling catheter in tethered animals. Cell counts (blood smears and stain) and ADCC assays were performed on samples of 10 ml heparinized blood drawn on selected days over a twenty day period.

## EXAMPLE II

## In Vitro Administration with In Vitro Testing of NK-ADCC Augmentation

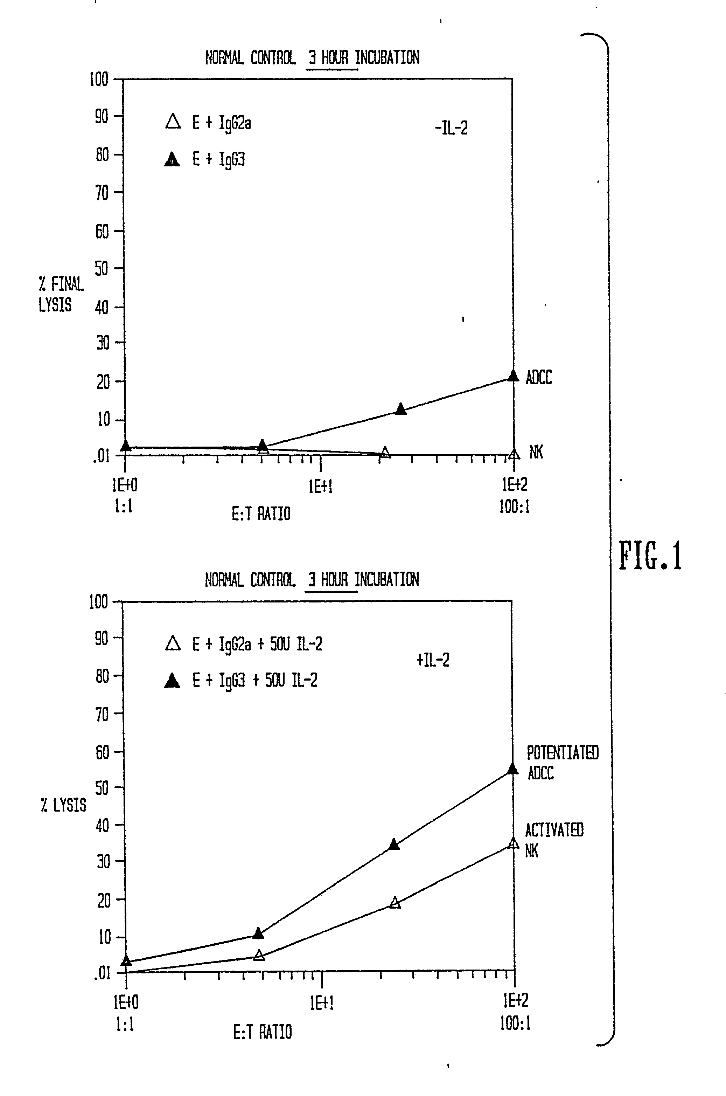

Normal donor lymphocytes were incubated in the presence or absence of 50 U/ml IL-2 for 3 h and then tested for cytotoxicity to $^{51}$Cr-labeled target cells. Figure 1 shows that, in the absence of IL-2, effector cells plus a murine IgG$_{2a}$ MAb (non-ADCC) did not lyse target cells. The level of killing was equal to killing with effector cells only. On the absence of IL-2, effector cells plus an IgG$_3$ MAb (ADCC) produced some target cell killing, particularly at higher E:T ratios. When normal donor lymphocytes were activated in the presence of IL-2, both killing with effector cells only and ADCC cytolysis were enhanced. The enhanced level of non-antibody-mediated killing will be referred to hereinafter as "activated NK killing." The "NK" designation reflects the fact that "LAK killing" requires more prolonged exposure of effector cells to higher doses of IL-2 to achieve optimal activation. While short exposure of effector cells to IL-2 augments both NK-ADCC and activated NK cytotoxic reactions, ADCC cytolysis under these experimental conditions is significantly greater than activated NK killing of target cells.

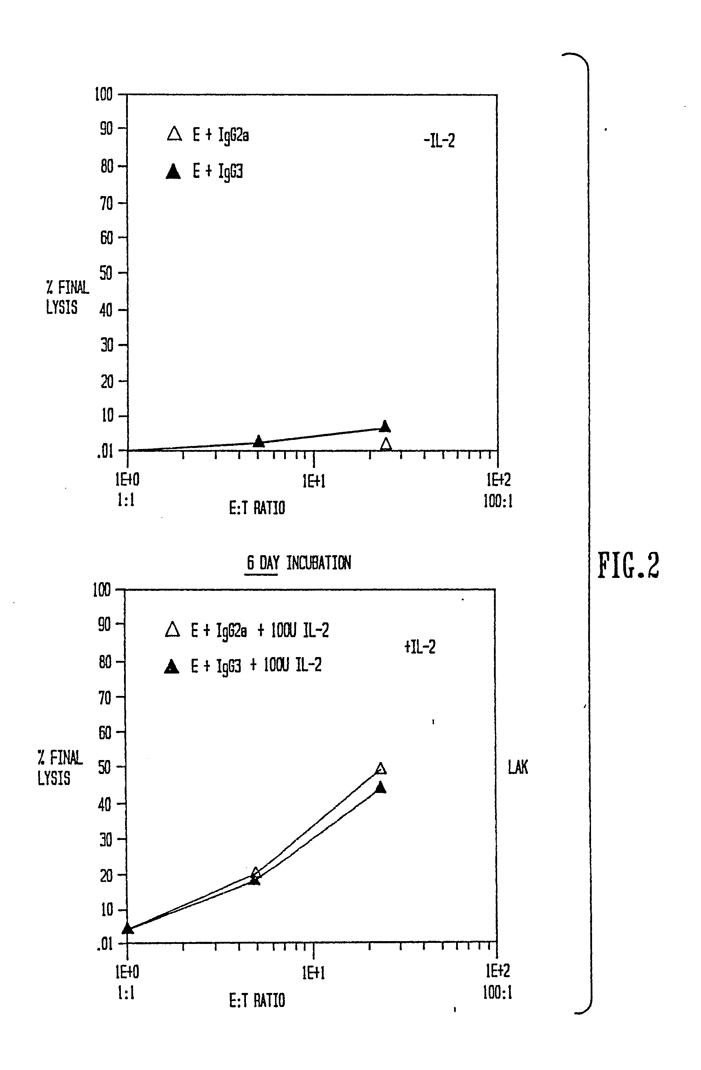

NK-ADCC was also tested with effector cells elicited with conditions typical for LAK cell generation (Figure 2). Normal donor lymphocytes were incubated in the presence or absence of 100 U/ml of IL-2 for 6 days prior to assay of cytotoxic activity. In the absence of IL-2, negligible NK-ADCC and LAK killing was observed. In the presence of IL-2, the cytolytic profiles for effector cells in the presence or absence of an ADCC-mediating MAb were essentially the same. These data indicate that the addition of MAb to the cytotoxicity assay does not provide additive killing effects. That is, no augmented NK-ADCC response occurs if the conditions used are optimal for LAK cell generation. Therefore, current clinical protocols designated to optimize LAK cell therapy would not be optimal for augmenting a patient's effector cells for an optimal NK-ADCC response.

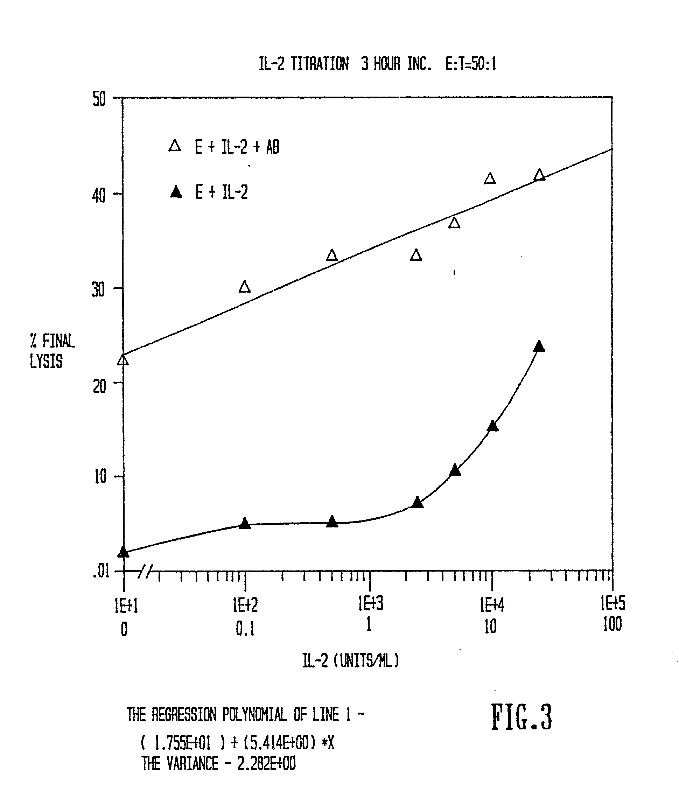

Titration of IL-2 demonstrated that the concentration of IL-2 required for augmentation of NK-ADCC is lower than the concentration of IL-2 required for activating NK killing (Figure 3) or, for that matter, LAK cell killing (see Figure 2). A short exposure (3 h) of effector cells to concentrations of 0.1 to 1.0 U/ml of IL-2 provided significant augmentation of ADCC without appreciable boosting of NK killing. Higher concentrations of IL-2 (10-50 U/ml) were needed for boosting of NK killing. In contrast, LAK cells are optimally generated in vivo by 3-6 days exposure of progenitor cells to 100-1000 U/ml IL-2 (J.H. Phillips and L.L. Lanier, J. Exp. Med. 164: 814, 1986). These data predict that NK-ADCC augmentation in vivo could be achieved with administration of lower doses of IL-2 than are required for activating NK or generating LAK cell killing.


## EXAMPLE III


### In Vivo Administration and Ex Vivo Testing of NK-ADCC Augmentation


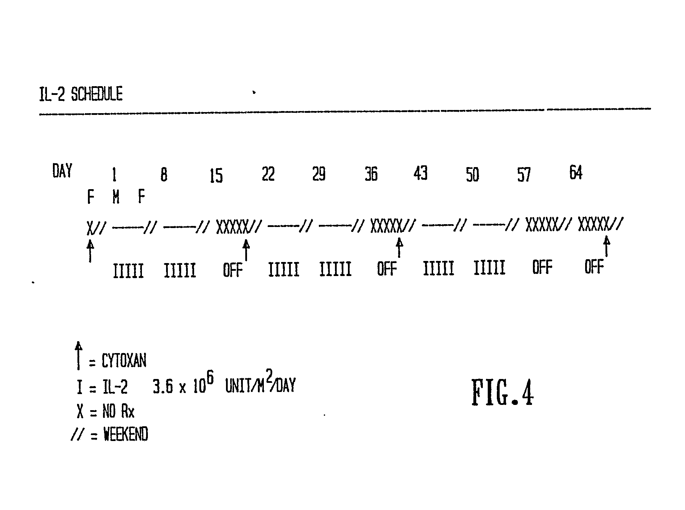

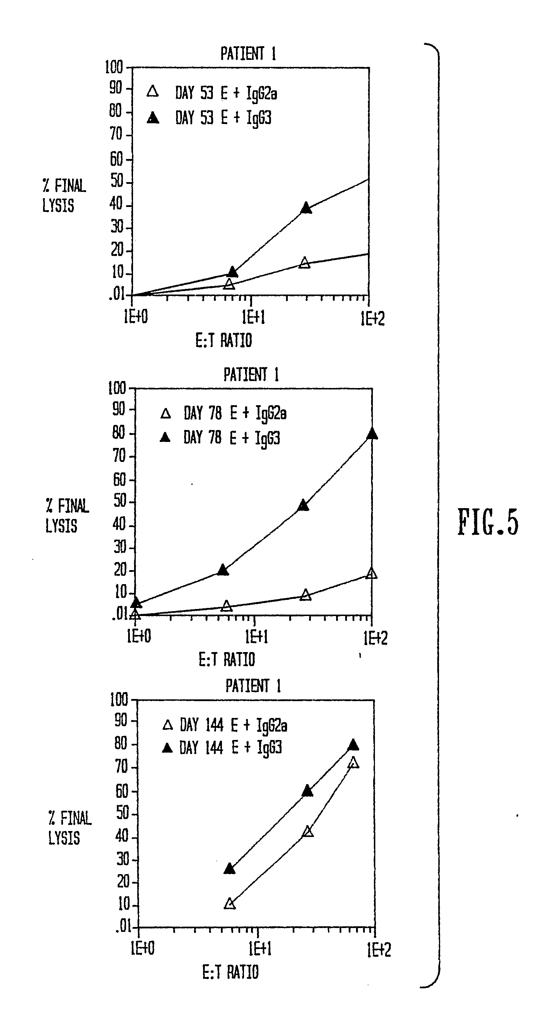

Shown in Figure 4 is a clinical protocol useful to achieve anti-tumor activity with IL-2 alone by abrogating immunosuppressive activity of T suppressor cells with cytoxan. The cytoxan pretreatment allows further expansion of LAK cell populations as their proliferation or activation is presumably not down-regulated by suppressor cells. "OFF" indicates that no IL-2 was administered. Prior to IL-2 therapy, patient lymphocytes showed similar or less ADCC killing with an IgG$_3$ MAb than normal donor lymphocytes that had been unstimulated with IL-2. Figure 5 shows the in vivo augmentation of one patient's NK-ADCC response observed after infusion of IL-2. The patient received IL-2 according to the protocol of Figure 4. Lymphocytes were removed from the patient at days 53, 78 and 144, and were assayed for NK-ADCC activity either alone or in the presence of an IgG$_{2a}$ MAb (non-ADCC) or an IgG$_3$ MAb (ADCC). The patient's lymphocytes exhibited pronounced augmentation of NK-ADCC, with greatest augmentation seen during the later stages of IL-2 administration. However, by day 144, elevated levels of LAK cell killing were apparent, and like the data reported in Example II, these in vivo-generated LAK cells did not mediate NK-ADCC killing of target cells. Based on this monitoring, patients would have most benefited from antibody administration in a period near the 78th day of IL-2 infusion. In order to avoid the loss of NK-ADCC augmentation by day 144, the patient could have had the levels of IL-2 reduced or the number of days of treatment reduced. Five additional patients receiving continuous infusions of IL-2 were monitored for NK-ADCC activity, and all showed augmentation of NK-ADCC that was significantly greater than levels of NK-ADCC achieved with their own unstimulated lymphocytes or normal donor lymphocytes.

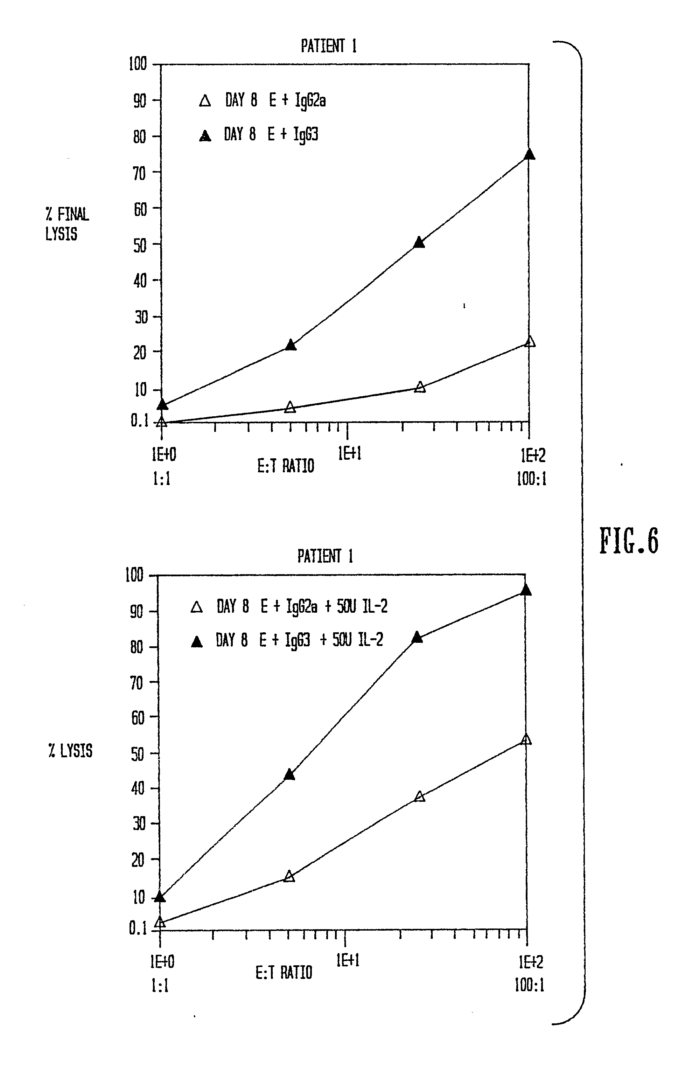

As an example, the maximal NK-ADCC response which can be achieved can be determined as was done for a day-8 sample from a patient receiving IL-2 therapy (Figure 6) . This estimation was made by adding exogenous IL-2 in vitro to a portion of the patient's already stimulated lymphocytes and comparing NK-ADCC activity in the presence or absence of the added IL-2. The addition of exogenous IL-2 increased both NK-ADCC and activated NK cell killing, indicating that the in vivo infusion of IL-2 over an 8-day treatment course had not resulted in maximal NK-ADCC activation. Dat of this type are used to justify further treatment of the patient by either increasing the dose of lymphokine or increasing the number of treatment days in order to further augment NK-ADCC activity prior to administration of antibody.

The data obtained from infusion of these tolerable levels of IL-2 suggest that optimal NK-ADCC responses may be achieved in vivo by continuous infusion of the lymphokine. The levels of circulating IL-2 required for augmentation of NK-ADCC responses in vivo, however, would not necessarily be optimal for activated NK or LAK killing of tumor target cells. In fact, the higher doses of IL-2 required for optimization of non-antibody-directed killing would likely abrogate the NK-ADCC augmentation achieved in earlier treatment.

## EXAMPLE IV

## PMN-ADCC in the Presence and Absence of GM-CSF

The superiority of murine $IgG_3$ monoclonal antibodies for mediation of ADCC with large granular lymphocytes (LGLs) is disclosed in a pending patent application. U.S. Serial No. 080,319, submitted by A.C. Morgan, Jr. The present study was performed to determine whether murine $IgG_3$ monoclonal antibodies are also effective mediators of ADCC with PMNs.

Monoclonal antibody NR-CO-04 ($IgG_3$) was added to human peripheral PMNs and LS-180 target cells in a standard 3 h $^{51}Cr$ release assay. Figure 1 shows that NR-CO-04 antibody alone was generally a poor mediator of ADCC with PMNs at effector to target ratios as great as 100:1.

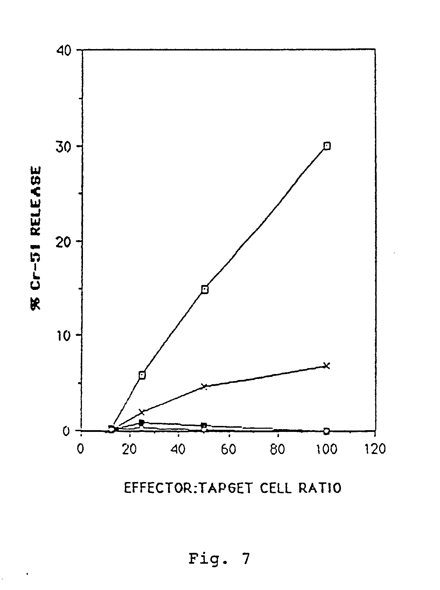

Figure 7 further illustrates that preincubation of PMNs with GM-CSF (500 U/ml) for 1 h before the addition of NR-CO-04 antibody and LS-180 target cells resulted in significant enhancement of PMN-$IgG_3$ ADCC. An $IgG_1$ monoclonal antibody (NR-CO-02) was completely ineffective as a mediator of PMN-ADCC with or without preincubation of the effector PMNs with GM-CSF.

GM-CSF augmentation of PMN-ADCC mediated by $IgG_3$ monoclonal antibodies is quantitated in Table 1.

TABLE 1

| Augmentation of PMN-Mediated $IgG_3$-ADCC by GM-CSF | | | |
|---|---|---|---|
| | Percent Cytotoxicity | | |
| | NSC[1] | ADCC | GM-CSF-ADCC[2] |
| Range | 0.4 - 9.9 | 2.2 - 18.4 | 16.2 - 39.7 |
| Mean | 1.6 | 8.0 | 26.9 |
| Standard deviation | 2.6 | 5.4 | 9.7 |
| N = 15 donors | | | |

[1] Nonspecific cellular cytotoxicity (NSC) mediated by PMNs in the absence of antibody.
2 Effector cells were incubated for 1 h in the presence of 100 U/ml GM-CSF before the addition of NR-CO-04 antibody and $^{51}CR$-labeled LS-180 target cells.

Analysis of PMN-mediated $IgG_3$ ADCC using PMNs obtained from 15 normal donors revealed that the mean ADCC value with unstimulated PMN was 8.0% specific release at an effector:target ratio of 50:1. When PMNs were preincubated for 1 h with GM-CSF at 100 U/ml, PMN-$IgG_3$ DCC activity increased to a mean value of 26.9% specific release.

Other potential activators of PMNs were examined for their ability to augment PMN-ADCC with $IgG_3$ monoclonal antibodies.

TABLE 2

| Effect of Various Activators on PMN-Mediated ADCC | | |
|---|---|---|
| Treatment[1] | Antibody | % Cytotoxicity[1] |
| GM-CSF, 30 min[2] | NR-CO-04 | 34.4 ± 2.7 |
| (100 U/ml) | NR-CO-02 | 2.0 ± 1.6 |
| | NR-LU-10 | 2.1 ± 0.6 |
| | None | 2.3 ± 0.6 |
| GM-CSF, 18 h | NR-CO-04 | |
| 100 U/ml | | 18.2 ± 0.8 |
| 10 U/ml | | 19.2 ± 1.8 |
| 1 U/ml | | 15.9 ± 0.4 |
| g-IFN, 30 min | NR-CO-04 | |
| 100 U/ml | | 26.9 ± 2.6 |
| 10 U/ml | | 23.1 ± 3.1 |
| 1 U/ml | | 12.1 ± 2.5 |
| g-IFN, 18 h | NR-CO-04 | |
| 100 U/ml | | 18.0 ± 0.7 |
| 10 U/ml | | 13.9 ± 2.0 |
| 1 U/ml | | 9.6 ± 0.9 |
| fMLP, 30 min | NR-CO-04 | |
| $10^{-7}$ M | | 16.2 ± 0.8 |
| $10^{-8}$ M | | 23.7 ± 1.2 |
| $10^{-9}$ M | | 15.6 ± 0.8 |
| PMA, 30 min | NR-CO-04 | 18.8 ± 0.6 |
| PMA + A23187, 30 min | NR-CO-04 | 17.4 ± 1.5 |
| M-CSF, 30 min | NR-CO-04 | |
| $1:10^{3}$ | | 2.3 ± 0.4 |
| $1:10^{4}$ | | 2.4 ± 1.2 |
| $1:10^{5}$ | | 2.3 ± 1.4 |

1 Effector to target cell ratio was 100:1 for a 3 h ADCC assay.

2 PMNs were pretreated with various activating agents for either 30 min or 18 h before adding LS-180 target cells and either NR-CO-04, NR-CO-02 or NR-LU-10 antibody.

Table 2 demonstrates that potential activators, such as gamma-IFN, fMLP, PMA, or PMA plus the calcium ionophore A23187, were less effective than GM-CSF for augmentation of PMN-mediated IgG$_3$ ADCC. As expected, monocyte colony stimulating factor (M-CSF) was completely ineffective for augmenting PMN-ADCC with NR-CO-04. When an IgG$_1$ monoclonal antibody (NR-CO-02) and an IgG$_{2b}$ monoclonal antibody (NR-LU-10; effective for augmenting LGL-ADCC) were tested with GM-CSF-activated PMNs, no augmentation of PMN-ADCC was seen.

In addition to a 30 min preincubation period with GM-CSF and gamma-IFN, PMNs were also pretreated with GM-CSF and gamma-IFN for 18h. Augmented ADCC was greater with GM-CSF preincubation than with gamma-IFN preincubation. However, the loss of PMN activation following the 18 h preincubation period was greater with GM-CSF-treated cells than with the gamma-IFN-treated PMNs, suggesting a protective effect of gamma-IFN.

EXAMPLE V

Antigen Specificity of GM-CSF-Augmented PMN-IgG3 ADCC

In order to characterize antigen specificity of GM-CSF-augmented PMN-IgG3 ADCC, NR-CO-04 and NR-CO-02 antibodies were tested (with and without GM-CSF preincubation) for targeting of carcinoma cell lines of both colon and breast origin.

TABLE 3

| Effect of GM-CSF on PMN-ADCC of Different Cell Lines | | | | | |
|---|---|---|---|---|---|
| Target Cell | Tumor Type | GM-CSF[1] | Antibody | FE Ratio | % Release[2] |
| LS-180(T) | Colon | + | NR-CO-04 |  | 21.2 ± 4.1 |
|  |  | - |  | 7.1 | 9.2 ± 2.1 |
|  |  | + | NR-CO-02 |  | 0.0 ± 2.2 |
|  |  | - |  | 2.0 | 0.0 ± 1.1 |
| LS-180 | Colon | + | NR-CO-04 |  | 42.5 ± 4.2 |
|  |  | - |  | 23.4 | 18.3 ± 1.6 |
|  |  | + | NR-CO-02 |  | 0.4 ± 0.6 |
|  |  | - |  | N.D. | 0.0 ± 0.2 |
| LS-174 | Colon | + | NR-CO-04 |  | 31.2 ± 1.5 |
|  |  | - |  | 5.0 | 11.4 ± 1.4 |
|  |  | + | NR-CO-02 |  | 2.7 ± 0.7 |
|  |  | - |  | N.D. | 1.3 ± 0.7 |
| SW1-222 | Colon | + | NR-CO-04 |  | 15.3 ± 2.9 |
|  |  | - |  | 17.3 | 1.7 ± 1.5 |
|  |  | + | NR-CO-02 |  | 10.3 ± 1.9 |
|  |  | - |  | 7.0 | 0.0 ± 1.6 |
| LoVo | Colon | + | NR-CO-04 |  | 2.9 ± 1.3 |
|  |  | - |  | 3.2 | 2.1 ± 1.1 |
|  |  | + | NR-CO-02 |  | 0.5 ± 1.0 |
|  |  | - |  | 7.3 | 0.0 ± 1.0 |
| MDA-MB-484 | Breast | + | NR-CO-04 |  | 23.3 ± 2.6 |
|  |  | - |  | 20.7 | 7.1 ± 1.9 |
|  |  | + | NR-CO-02 |  | 0.0 ± 0.4 |
|  |  | - |  | 6.5 | 0.0 ± 2.0 |

[1] PMNs were preincubated with GM-CSF for 30 min before addition of the various $^{51}$Cr-labeled target cells and either IgC3 or IgG1 antibody.

[2] Effector to target cell ratio was 100:1 for a 3 h ADCC assay.

Table 3 demonstrates that NR-CO-04, but not NR-CO-02, monoclonal antibody mediated substantial PMN-ADCC at an effector:target ratio of 100:1 in the presence of GM-CSF only. Augmented PMN-IgG3 ADCC was observed with cell lines of either colon or breast origin. Table 3 further indicates that PMN-IgG3 ADCC was effective against numerous antigen-positive target cells. The data also suggest good correlation between antigen density on target cells and cytotoxicity, as evidenced by the intensity of antibody labeling (F.E. ratio) and percent cytotoxicity.

Antigen-positive cells explanted in nude mice [LS-180(T)] displayed lower antigen density than the same cells cultured in vitro (LS-180), yet LS-180(T) target cells were susceptible to significant levels of PMN-ADCC. However, one cell line (LoVo) with low but significant levels of NR-CO-04 binding was not susceptible to PMN-ADCC.

EXAMPLE VI

18

Titration of GM-CSF for PMN-IgG₃ ADCC


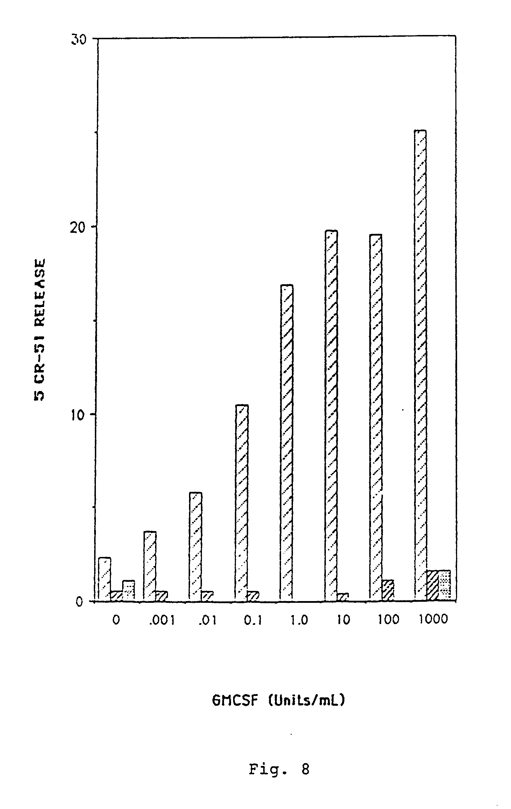

PMNs were preincubated for 1 h with dilutions of GM-CSF (starting with 1000 U/ml), in order to determine the concentrations of GM-CSF required for optimal augmentation of PMN-mediated ADCC. Figure 8 shows a dose-dependent augmentation of PMN-ADCC with NR-CO-04 antibody. Significant augmentation of PMN-ADCC was observed with concentrations of GM-CSF as low as 0.01 U/ml, and maximal $^{51}$Cr release was obtained with concentrations of GM-CSF greater than 10 U/ml. Augmentation was strictly dependent on the presence of the IgG₃ NR-CO-04 antibody, as PMNs preincubated with GM-CSF at any concentration failed to mediate ADCC of LS-180 target cells either in the presence of NR-CO-02 (IgG₁) or in the absence of antibody.


## EXAMPLE VII


### Kinetics of GM-CSF augmentation of PMN-Mediated ADCC


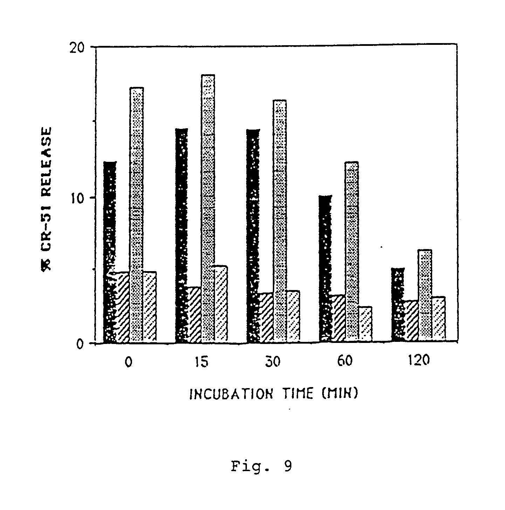

The kinetics of GM-CSF activation of PMNs for ADCC was examined by exposing PMNs to lymphokine for periods ranging from less than 5 min to 2 h. Figure 9 demonstrates that GM-CSF augmentation of PMN-IgG₃ ADCC is a very rapid event. Immediate washing of the exposed PMNs followed by centrifugation results in nearly optimal levels of ADCC. Longer periods of exposure to GM-CSF (2 h) resulted in a decreased augmentation of PMN-ADCC approaching non-stimulated levels.


## EXAMPLE VIII


### Comparison with Protein Kinase Activation of Effectors


Protein kinase C-mediation of neutrophil natural cytotoxicity against K562 target cells has been reported by R. Gavioli et al., Biochem. Biophys. Res. Comm. 148: 1290-94, 1987. To determine whether GM-CSF activation of PMNs acts in a similar manner, the effects of protein kinase C inhibitors and activators was examined. Retinal, a protein kinase C inhibitor, abrogated GM-CSF augmentation of PMN-ADCC in a dose-dependent fashion, as shown in Table 4.

## TABLE 4

### Effect of Retinal and PMA on
### GM-CSF Augmentation of PMN-ADCC

| Retinal, $\mu M$[1] | GM-CSF (10 U/ml) | % $^{51}$Cr Release | % Control Release |
|---|---|---|---|
| 0.0 | − | 14.6 ± 1.7 | − |
|  | + | 35.1 ± 0.9 | − |
| 1.0 | − | 8.9 ± 1.6 | 60 |
|  | + | 29.4 ± 1.9 | 85 |
| 10.0 | − | 7.9 ± 1.6 | 54 |
|  | + | 25.0 ± 0.0 | 71 |
| 100.0 | − | 7.6 ± 2.5 | 52 |
|  | + | 19.1 ± 0.6 | 54 |
| Retinal, $\mu M$ + PMA + A23187[2] |  |  |  |
| 0.0 | − | 20.0 ± 1.6 | − |
|  | + | 30.5 ± 4.3 | − |
| 1.0 | − | 22.8 ± 1.8 | 114 |
|  | + | 33.4 ± 0.6 | 110 |
| 10.0 | − | 21.0 ± 5.3 | 105 |
|  | + | 31.5 ± 3.0 | 103 |
| 100.0 | − | 19.8 ± 0.3 | 99 |
|  | + | 42.8 ± 4.7 | 140 |

[1] PMN were incubated for 30 min in the absence of retinal or in the presence of 1 to 100 $\mu m$ retinal, followed by a 15 min incubation with or without added GM-CSF. NR-CO-04 antibody and LS-180 target cells were then added for a 3 h ADCC assay.

[2] PMN were treated as above, except that PMA and A23187 were added immediately before the NR-CO-04 antibody and LS-180 target cells.

Treatment of PMNs with GM-CSF failed to reverse inhibition by retinal. However, the addition of PMA, a protein kinase C activator, and the calcium ionophore A23187 prior to the addition of retinal restored PMN-ADCC to augmented levels. The low level of PMN-ADCC observed in the absence of GM-CSF stimulation was further depressed by retinal in a dose-dependent fashion.

## EXAMPLE IX

### Ex Vivo Analysis of PMN-ADCC in Monkeys Infused with GM-CSF

In vivo exposure of PMNs to GM-CSF induces leukocytosis of monkey PMNs and eosinophils (P. Mayer et al., Blood 70:206-13, 1987). The preceding examples describe the potent in vitro effect of GM-CSF on PMN-ADCC. Therefore, monkeys were infused with GM-CSF and tested for in vivo augmentation of PMN-ADCC.

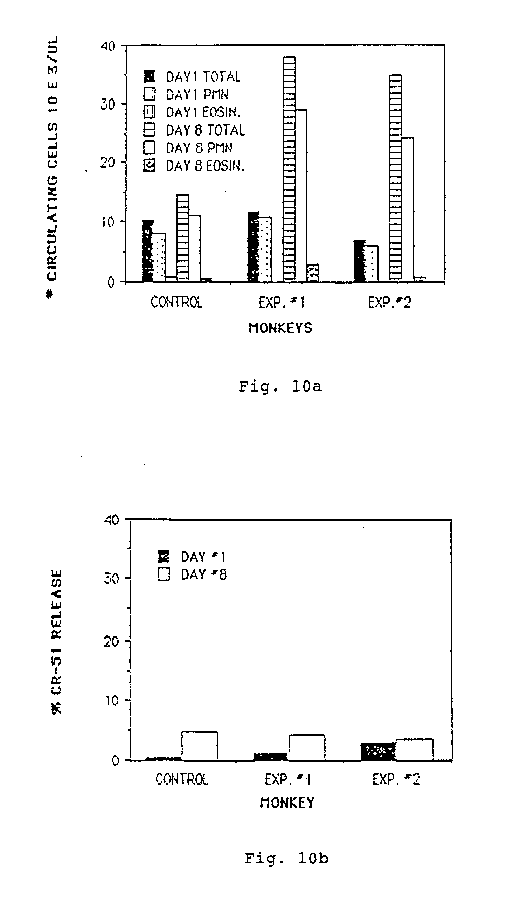

M. fasicularis monkeys were infused continuously with GM-CSF over a period of 2 weeks. At various time points, blood samples were withdrawn, lymphocytes were counted, and ADCC activity was assessed. Figure 10 demonstrates that GM-CSF significantly enhanced the number of circulating total leukocytes, the majority of which were neutrophils, after 8 days of infusion.

Ex vivo analysis of the effector function of mature PMNs after 8 days of GM-CSF infusion revealed that NR-CO-04-mediated ADCC activity against LS-180 target cells was not significantly enhanced in the GM-CSF-treated monkeys, as compared to the untreated control. Longer periods of infusion with GM-CSF did not improve the numbers of circulating cells nor PMN-ADCC activity (data not shown). Other studies have shown that short term (1 h) exposure of monkey PMNs to GM-CSF resulted in significant levels of ADCC against LS-180 target cells (data not shown). These data suggest that while GM-CSF may act as an effective in vivo colony stimulating factor, GM-CSF alone, under the conditions tested, is a poor inducer of ADCC activity in vivo.

## EXAMPLE X

### Ex Vivo Activation of PMN with GM-CSF For Augmentation of ADCC in a Tumor-Bearing Patient

Cytophoresis is performed on a tumor-bearing patient, in order to obtain from peripheral blood a fraction enriched for mature PMNs. Briefly, the PMN enrichment technique involves standard blood phoresis performed in combination with hydroxyethyl starch, a sedimenting agent. The patient may also be pretreated with prednisone for 12-18 h immediately preceding the phoresis process. Prednisone is a steroid that induces release of mature neutrophils from the bone marrow to the peripheral blood. The PMNs are collected under sterile conditions, with a typical cellular recovery approximating $30 \times 10^9$ cells/cytophoresis process.

The PMNs are incubated for 15-30 min with 100 U/ml GM-CSF, in order to generate "activated PMNs." Alternatively, the patient's PMNs may be activated with a combination of GM-CSF and gamma-IFN overnight. For instance, when a combination of lymphokines is used, PMNs may be incubated with 100 U/ml GM-CSF and an ADCC-stimulatory amount of gamma-IFN simultaneously, or with an ADCC-stimulatory amount of gamma-IFN prior to incubation of the PMNs with 100 U/ml GM-CSF for 15 min.

Immediately after cytophoresis of the patient is completed, and before reinfusion of activated PMNs, murine IgG₃ monoclonal antibody NR-CO-04 is administered to the patient. The amount of ADCC-effective monoclonal antibody infused will range from about 10 mg/70 kg to about 500 mg/70 kg. Ideally, administration of monoclonal antibody is completed 24-72 h before reinfusion of activated PMNs. At this time, serum levels of antibody will be reduced and optimal levels of antibody will be bound to the tumor cells. The activated PMNs are then reinfused into the tumor-bearing patient along with an additional amount of GM-CSF.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

The features disclosed in the foregoing description, in the claims and/or in the accompanying drawings may, both separately and in any combination thereof, be material for realising the invention in diverse forms thereof.

Claims

1. A method of in vitro assay for determining a time of optimal in vivo augmentation of an antibody-dependent, cell-mediated cytotoxicity (ADCC) response in a tumor-bearing patient, comprising the steps of:
determining a basal level of ADCC and NK or LAK cell activity, utilizing a basal effector cell sample from a patient;
assaying at each interval a first aliquot of effector cells removed from the patient at a predetermined interval after administration of a single or a plurality of doses of one or more lymphokines to the patient to establish a test level of ADCC above the basal level;
assaying at each interval a second aliquot of the effector cells to determine the change in NK cell activity above the basal level, thereby establishing a level of activated NK activity; and
determining a time of optimal in vivo augmentation of the effector cells for an ADCC response.

2. The method of claim 1 wherein the basal effector cell sample comprises multiple samples removed over a period of time.

3. The method of claim 1 or 2 wherein the step of determining the basal level of ADCC comprises the steps of:
combining the basal effector cell sample, an antibody, and target cells; and
detecting cytotoxicity by a target cell lysis assay.

4. The method of claim 1 or 2 wherein the step of assaying the first and subsequent aliquots of effector cells comprises the steps of:
combining the aliquot of effector cells with an antibody, and target cells; and
detecting cytotoxicity with a target cell lysis assay.

5. The method of claim 3 or 4 wherein the antibody is a monoclonal antibody.

6. The method of claim 3, 4 or 5 wherein the antibody is selected from murine IgG$_3$, rat IgG$_{2b}$, chimeric (mouse/human) IgG$_1$, chimeric IgG$_2$, chimeric IgG$_3$, chimeric IgG$_4$, and heterohybrid monoclonal antibodies capable of bi-arm binding.

7. The method of any one of claims 3 to 6 wherein the target cell lysis assay is selected from $^{51}$Chromium release, $^{111}$Indium release, and trypan blue exclusion.

8. The method of claim 3 or 4 wherein the target cells are tumor cells from the patient.

9. The method of claim 3 or 4 wherein the target cells are cultured tumor cells that are antigenically similar to tumor cells from the patient.

10. The method of claim 1 or 2 wherein the step of determining the basal level of ADCC comprises the steps of:
incubating the basal effector cell sample with a first and second labeled antibody having specificity for a cell surface or intracellular marker associated with activated effector cells; and
measuring a level of effector cell activation using two color fluorescence cytometry.

11. The method of claim 1 or 2 wherein the step of assaying the first and subsequent aliquots of effector cells comprises the steps of:
incubating the aliquot of effector cells with a first and second labeled antibody having specificity for a cell surface or intracellular marker associated with activated effector cells; and
measuring a level of effector cell activation using two color fluorescence cytometry.

12. The method of claim 10 or 11 wherein the antibody is NKH-1, OKM-1, anti-LFA-1, anti-CD-2 or anti-LFA-3.

13. The method of any one of claims 1-12 wherein the lymphokine or combination of lymphokines is selected from interleukin-2, interleukin-4, interleukin-6, GM-CSF, G-CSF, M-CSF, and interleukin-3.

14. The method of claim 13 wherein the lymphokine combination is interleukin-2 and interleukin-4.

15. The method of claim 13 wherein the lymphokine combination is interleukin-2 and interleukin-6.

22

16. The method of any one of claims 1-12 wherein the effector cells are characterized by Leu-M1 or OKM-1 cell surface markers or the LGL phenotype.

17. The method of claim 1 wherein the step of determining a time of optimal activation comprises the steps of:

comparing, at each time interval, the test level of ADCC with or without exogenous lymphokine and the test level of LAK or NK activity to determine a difference between the same test levels; and

identifying the time at which no further augmentation of ADCC occurs by addition of exogenous lymphokine or lymphokine combinations.

18. The method of claim 17 wherein the preselected differences are calculated by lytic units.

19. The method of any one of claims 1-18 wherein cytoxan had been administered to the patient before or during lymphokine administration.

20. The method of claim 19 wherein the cytoxan had been administered intravenously at least 24 hours before IL-2 administration.

21. The method of any one of claims 1-20, wherein the lymphokine had been infused in dosages adequate to maintain the optimal in vivo activation of the effector cells for a predetermined period of time after the step of determining a time of optimal in vivo activation of the effector cells for an ADCC response.

22. A method for ex vivo activation of polymorphonuclear leukocytes (PMNs) for use in antibody-dependent cellular cytotoxicity (ADCC) therapy mediated by monoclonal antibody, comprising the steps of:

separating a polymorphonuclear leukocyte (PMN) fraction from a leukocyte-containing sample removed from an appropriate donor; and

incubating the PMN fraction with an ADCC-stimulatory amount of activator, thereby producing activated PMNs for use in ADCC therapy mediated by monoclonal antibody.

23. The method of claim 22 wherein the leukocyte-containing sample is blood.

24. The method of claim 22 wherein the leukocyte-containing sample is bone marrow.

25. The method of claim 22, 23 or 24 wherein the activator is a lymphokine.

26. The method of claim 22, 23 or 24 wherein the activator is a protein kinase activator.

27. The method of claim 22, 23 or 24 wherein the activator is selected from GM-CSF, G-CSF, gamma-IFN, fMLP, and PMA.

28. The method of claim 22, 23 or 24 wherein the activator is a combination of lymphokines, a combination of protein kinase activators, or a combination of lymphokines and protein kinase activators.

29. The method of claim 28 wherein the combination of lymphokines is GM-CSF and gamma-IFN.

30. The method of claim 22 wherein the ADCC-stimulatory amount of activator is from about 50 U/ml to about 1000 U/ml.

31. The method of claim 30 wherein the ADCC-stimulatory amount of activator is from about 100 U/ml to about 500 U/ml.

32. The method of any one of claims 22 to 31 wherein the ADCC response is measured using a target cell lysis assay selected from $^{51}$Chromium release, $^{111}$Indium release and trypan blue exclusion.

33. The method of any one of claims 22 to 31 wherein the ADCC response is measured using two color fluorescence cytometry in combination with a first and second labeled antibody having specificity for a cell surface or intracellular marker associated with activated effector cells.

34. The method of claim 33 wherein the antibody is Leu-M1, OKM-1, anti-LFA-1, anti-CD-2 or anti-LFA-3.

35. The method of claim 22 further comprising, after the step of incubating, sensitizing the activated PMNs with an ADCC-effective amount of a monoclonal antibody directed against the patient's tumor, thereby forming an activated PMN-monoclonal antibody complex.

36. The method of claim 35 wherein the monoclonal antibody is selected from murine IgG$_3$, rat IgG$_{2b}$, chimeric (mouse/human) IgG$_1$, chimeric IgG$_3$, and heterohybrid monoclonal antibodies capable of bi-arm binding to both target cells and activated PMN effector cells.

37. The method of claim 35 wherein the monoclonal antibody is an anti-carcinoma monoclonal antibody.

38. The method of claim 37 wherein the anti-carcinoma monoclonal antibody is NR-CO-04.

39. The method of claim 35 wherein the monoclonal antibody is coupled to a chemotactic agent.

40. The method of claim 39 wherein the chemotactic agent is fMLP.

41. A method for enhanced ex vivo activation of polymorphonuclear leukocytes (PMNs) for use in antibody-dependent cellular cytotoxicity (ADCC) therapy mediated by monoclonal antibody, comprising the steps of:

separating a polymorphonuclear leukocyte (PMN) fraction from a leukocyte-containing sample removed from a tumor-bearing patient into whom had been infused a first activator in an amount sufficient for in vivo

23

proliferation of PMN effector cells, thereby inducing leukocytosis in the patient; and

incubating the PMN fraction with an ADCC-stimulatory amount of a second activator, thereby producing activated PMNs for use in ADCC therapy mediated by monoclonal antibody.

42. The method of claim 41 wherein the leukocyte-containing sample is blood.

43. The method of claim 41 wherein the leukocyte-containing sample is bone marrow.

44. The method of claim 41, 42 or 43 wherein the first or second activator is a lymphokine.

45. The method of claim 41, 42 or 43 wherein the first or second activator is a protein kinase activator.

46. The method of claim 41, 42 or 43 wherein the first or second activator is selected from GM-CSF, G-CSF, gamma-IFN, fMLP,and PMA.

47. The method of claim 41, 42 or 43 wherein the first or second activator is a combination of lymphokines, a combination of protein kinase activators, or a combination of lymphokines and protein kinase activators.

48. The method of claim 47 wherein the combination of lymphokines is GM-CSF and gamma-IFN.

49. The method of claim 41 wherein the ADCC-stimulatory amount of the second activator is from about 50 U/ml to about 1000 U/ml.

50. The method of claim 49 wherein the ADCC-stimulatory amount of the second activator is from about 100 U/ml to about 500 U/ml.

51. The method of any one of claims 41 to 50 wherein the ADCC response is measured using a target cell lysis assay selected from [5']Chromium release, [111]Indium release and trypan blue exclusion.

52. The method of any one of claims 41 to 50 wherein the ADCC response is measured using two color fluorescence cytometry in combination with a first and second labeled antibody having specificity for a cell surface or intracellular marker associated with activated effector cells.

53. The method of claim 52 wherein the antibody is Leu-M1, OKM-1, anti-LFA-1, anti-CD-2 or anti-LFA-3.

54. The method of claim 41 further comprising, after the step of incubating, removing the second activator from the activated PMN effector cells.

55. The method of claim 41 further comprising, after the step of incubating, sensitizing the activated PMNs with an ADCC-effective amount of a monoclonal antibody directed against the patient's tumor, thereby forming an activated PMN-monoclonal antibody complex.

56. The method of claim 55 wherein the monoclonal antibody is selected from murine $IgG_3$, rat $IgG_{2b}$, chimeric (mouse/human) $IgG_1$, chimeric $IgG_3$, and heterohybrid monoclonal antibodies capable of bi-arm binding to both target cells and activated PMN effector cells.

57. The method of claim 55 wherein the monoclonal antibody is an anti-carcinoma monoclonal antibody.

58. The method of claim 57 wherein the anti-carcinoma monoclonal antibody is NR-CO-04.

59. The method of claim 55 wherein the monoclonal antibody is coupled to a chemotactic agent.

60. The method of claim 59 wherein the chemotactic agent is fMLP.

61. The use of an effective amount of one or more activators capable of stimulating effector cells for ADCC for the manufacture of a medicament for use in augmenting an in vivo ADCC response in a tumor-bearing patient.

62. The use of claim 61 wherein the activator is a lymphokine.

63. The use of claim 61 wherein the activator is a protein kinase activator.

64. The use of claim 61 wherein the activator is selected from interleukin-2, interleukin-4, interleukin-6, GM-CSF, G-CSF, M-CSF, interleukin-3, gamma-IFN, fMLP, and PMA.

65. The use of claim 61 wherein the activator is a combination of lymphokines, a combination of protein kinase activators, or a combination of lymphokines and protein kinase activators.

66. The use of claim 65 wherein the lymphokine combination is interleukin-2 and interleukin-4.

67. The use of claim 65 wherein the lymphokine combination is interleukin-2 and interleukin-6.

68. The use of claim 65 wherein the lymphokine combination is GM-CSF and gamma-IFN.

24

NORMAL CONTROL  3 HOUR INCUBATION

FIG.1

NORMAL CONTROL  3 HOUR INCUBATION

FIG.2

6 DAY INCUBATION

IL-2 TITRATION  3 HOUR INC.  E:T=50:1

△  E + IL-2 + AB

▲  E + IL-2

% FINAL LYSIS

IL-2 (UNITS/ML)

THE REGRESSION POLYNOMIAL OF LINE 1 –

( 1.755E+01 ) + (5.414E+00) *X
THE VARIANCE – 2.282E+00

FIG.3

IL-2 SCHEDULE
_____

```
DAY     1      8      15      22      29      36      43      50      57      64
     F  M  F
     X// ----// ----// XXXXX// ----// ----// XXXXX// ----// ----// XXXXX// XXXXX//
     ↑              ↑                     ↑                            ↑
        IIIII  IIIII   OFF   IIIII  IIIII   OFF    IIIII  IIIII   OFF     OFF
```

↑ = CYTOXAN

I = IL-2    $3.6 \times 10^6$  UNIT/M$^2$/DAY          FIG.4

X = NO Rx

// = WEEKEND

EP 0 302 435 A1

FIG.5

FIG.6

Fig. 7

GMCSF (Units/mL)

Fig. 8

Fig. 9

Fig. 10a

Fig. 10b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 203 403 (ASAHI KASEI KOGYO K.K.) * Page 11, line 11 - page 12, line 17; claim 7 * | 1-4,8-10,13-15,17,18,41-44,64-67 | G 01 N 33/569 G 01 N 33/50 |
| X | US-A-4 677 061 (GENETIC SYSTEMS CORP.) * Column 1, lines 57-68; column 3, line 55 - column 4, line 20; column 9, lines 9-17 * | 1-6,10,56 | |
| Y | EP-A-0 227 335 (UNIVERSITY OF OTAGO) * Page 2, lines 15-23; page 4, line 7 - page 5, line 16; page 8, lines 17-31; page 9, lines 6-23; page 28, lines 1-13; figure 1 * | 1-6,8-10,19,20,56 | |
| Y | US-A-4 607 007 (BECTON DICKINSON & CO.) * Column 1, lines 38-68; column 2, line 39 - column 3, line 32; column 7, lines 5-26 * | 1-6,8-10,19,20,56 | TECHNICAL FIELDS SEARCHED (Int. Cl.4) G 01 N |
| X | SCIENCE, vol. 233, 19th September 1986, pages 1318-1321, Washington, D.C., US; S.A. ROSENBERG et al.: "A new approach to the adoptive immunotherapy of cancer with tumor-infiltrating lymphocytes" * Whole article * -/- | 1-4,8,9,19,20 | |

The present search report has been drawn up for all claims

| Place of search | Date of completion of the search | Examiner |
|---|---|---|
| THE HAGUE | 08-11-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF THE NATIONAL CANCER INSTITUTE, vol. 75, no. 4, October 1985, pages 595-603, Bethesda, MD, US; S.A. ROSENBERG: "Lymphokine-activated killer cells: a new approach to immunotherapy of cancer" * Whole article * | 1-4,8-10,13-15,17,18,41-44,64-67 | |
| X | ADVANCES IN CANCER RESEARCH, vol. 25, 1977, pages 323-389, US; S.A. ROSENBERG et al.: "Passive immunotherapy of cancer in animals and man" * Page 333, line 23 - page 337, line 7; page 347, line 13 - page 360, line 22; page 366, line 21 - page 369, line 34; page 374, line 16 - page 382, line 7 * | 1-4,8,9,19,20 | |
| X | RECENT ADVANCES IN CHEMOTHERAPY, INTERNATIONAL CONGRES ON CHEMOTHERAPY, vol. Anticancer, section 1, 1985, pages 75-82, US; R. LAFRENIERE et al.: "Lymphokine-activated killer cells and recombinant interleukin-2: a new approach to the immunotherapy of cancer" * Page 75, line 1 - page 79, line 17 * | 1-4,8-10,13-15,64 | TECHNICAL FIELDS SEARCHED (Int. Cl.4) |
| X | CHEMICAL ABSTRACTS, vol. 83, 1975, page 603, no. 6959e, Columbus, Ohio, US; C.J. SANDERSON et al.: "Kinetics of chromium-51 release from target cells in cell mediated cytotoxicity and the relation to the kinetics of killing", & CELL TISSUE KINET. 1975, 8(1), 23-32 * Abstract * -/- | 7 | |

The present search report has been drawn up for all claims

| Place of search | Date of completion of the search | Examiner |
|---|---|---|
| THE HAGUE | 08-11-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................
& : member of the same patent family, corresponding document

EPO FORM 1503 03.82 (P0401)

European Patent Office

**EUROPEAN SEARCH REPORT**

Application Number

EP 88 11 2480

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 106, 1987, page 441, no. 16664n, Columbus, Ohio, US; D. HASKARD et al.: "T lymphocyte adhesion to endothelial cells: mechanisms demonstrated by anti-LFA-1 monoclonal antibodies" * Abstract * | 12 | |

TECHNICAL FIELDS SEARCHED (Int. Cl.4)

The present search report has been drawn up for all claims

| Place of search | Date of completion of the search | Examiner |
|---|---|---|
| THE HAGUE | 08-11-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P0401)